# EUROPÄISCHE PATENTSCHRIFT

(11) **EP 1 572 226 B1**
(45) Veröffentlichungstag und Bekanntmachung des Hinweises auf die Patenterteilung: **09.07.2008**
(21) Anmeldenummer: 03813865.7
(22) Anmeldetag: 20.12.2003
(51) Int. Cl.: A61K 38/16

(54) **PEPTIDE MIT HOHEM CYSTEINGEHALT**
PEPTIDES HAVING A HIGH CYSTEINE CONTENT
PEPTIDES A HAUTE TENEUR EN CYSTEINE

(30) Priorität: 21.12.2002 DE 10260537
(43) Veröffentlichungstag der Anmeldung: 14.09.2005
(73) Patentinhaber: Kerek, Franz, 81375 München (DE)
(72) Erfinder: Kerek, Franz, 81375 München (DE)
(74) Vertreter: Arth, Hans-Lothar
(86) Internationale Anmeldenummer: PCT/DE2003/004228
(87) Internationale Veröffentlichungsnummer: WO 2004/058813

(56) Entgegenhaltungen:
- WO-A-02/22159
- DE-A- 4 208 923
- MILBRADT ALEXANDER G ET AL: "Structural characterization of hellethionins from Helleborus purpurascens." BIOCHEMISTRY, Bd. 42, Nr. 8, 4. März 2003 (2003-03-04), Seiten 2404-2411, XP002289555 ISSN: 0006-2960

## Beschreibung

### Technisches Gebiet

Die vorliegende Erfindung betrifft Peptide mit hohem Cysteingehalt, Nukleinsäuresequenzen, die diese Peptide codieren, Vektoren, die diese Sequenzen umfassen sowie pharmazeutische Zubereitungen enthaltend diese Peptide und deren Verwendung als Pharmazeutika.

### Stand der Technik

Als Pathogen wird ein Mikroorganismus (Bakterium, Virus, Hefe, etc.) oder ein übertragbares Agens (Toxin, etc.) bezeichnet, das eine Krankheit oder eine symptomlose Infektion eines pflanzlichen, tierischen oder menschlichen Organismus verursachen kann. Im weiteren Sinne werden auch Insekten oder Nematoden, die infektiöse Agenzien an Organismen übertragen können, als Pathogen bezeichnet. Gegen Angriffe von Pathogenen sind biologische Organismen mit einem komplexen und sehr effizienten Abwehrsystem ausgerüstet. Die Hauptaufgabe dieses Abwehr- bzw. Immunsystems ist es, das Eindringen der Pathogene zu verhindern bzw. die bereits eingedrungenen Pathogene unschädlich zu machen. Auch einfache Lebewesen verfügen diesbezüglich über mehrere Abwehrstoffe und Schutzmechanismen. Das Immunsystem von Vertebraten ist ein sehr komplexes Netzwerk von zellulären und humoralen Mechanismen mit der zusätzlichen Fähigkeit spezifische Antikörper gegen einzelne Pathogene bilden zu können und diese dadurch unschädlich zu machen.

Wenn die eigenen Abwehrkräfte des Organismus nicht ausreichen um das Pathogen unschädlich zu machen, müssen zusätzliche Hilfsmittel eingesetzt werden, wie z.B. Antibiotika, Fungizide, anti-virale und andere Agenzien. Ein ernsthaftes Problem dieser chemischen Pathogen-Bekämpfung ist jedoch die Tatsache, dass die Pathogenstämme gegen die eingesetzten Mittel sehr oft eine Resistenz erwerben. Diese Anpassung kann mit steigender Wirkstoffdosis temporär überwunden werden, aber auf lange Sicht werden immer neue chemische und biologische Mittel zur Pathogenbekämpfung benötigt.

Peptide sind aus Aminosäuren aufgebaute Verbindungen, die abhängig von der Zahl der enthaltenen Aminosäure-Einheiten als Di-, Tri-, Oligo- oder Polypeptide bezeichnet werden. Bei Proteinen handelt es sich um Polypeptide mit einer molaren Masse größer 10 kDa. Im Rahmen der vorliegenden Erfindung wird die Bezeichnung "Peptide" für Polypeptide mit einer molaren Masse kleiner als 10 kDa verwendet. Proteine bzw. Peptide sind an fast allen zellulären Prozessen maßgeblich beteiligt. Ihre zentrale Stellung in den Lebensvorgängen spiegelt sich darin wider, dass die genetische Information letztendlich in Form von Polypeptiden ausgedrückt wird.

Proteine werden anhand ihrer biologischen Aufgaben als Enzyme, Transport-, Struktur-, Abwehr-, Kontraktil-, Rezeptor-Proteine etc. bezeichnet. Bei Peptiden und bei einigen, meist kleineren Proteinen stehen andere biologische Aufgaben im Vordergrund wie die Hormon-Wirkung (z.B. Insulin, Oxytocin), Signalübertragung an Rezeptoren (z.B. Enkephaline), Steuerung der Immunabwehr (Zytokine) oder eine unspezifische Abwehr (z.B. Defensine). Viele biologisch hochwirksame Peptide werden in der Regel erst am Ort des Geschehens von größeren Vorstufenproteinen freigesetzt. Dadurch kann sich ihre Wirkung optimal entfalten.

Trotz ihrer hohen pharmakologischen Wirksamkeit, ihrer geringen Toxizität und ihrer guten Biokompatibilität ist die Verwendung von Peptiden als Arzneimittel sehr begrenzt. Ein grundsätzliches Hindernis besteht darin, dass Peptide im Organismus meistens sehr schnell abgebaut und dadurch unwirksam gemacht werden. Es wird seit längerem versucht diese schnelle Metabolisierung durch den Einbau von Hydrolyse-resistenten Strukturelementen wie z.B. D-Aminosäuren oder auf eine andere Art zu verhindern. Das Problem dabei ist, dass durch solche Strukturänderungen die therapeutische Wirksamkeit sehr stark beeinträchtigt wird.

Per oral eingenommene Proteine / Peptide werden im Magen-Darm-Trakt zersetzt und somit praktisch unwirksam gemacht. Dieser Verabreichungsweg ist für Peptid-Arzneistoffe also sehr problematisch. Die in der Therapie verwendeten körperidentischen Peptide wie Insulin, Erythropoietin, etc. werden aus diesem Grunde überwiegend (> 90%) nur parenteral (durch Injektionen) verabreicht. Ein weiteres technisches Problem für die Anwendung von größeren Peptiden ist deren Herstellung im industriellen Maßstab, die bisher nur in wenigen Fällen zufriedenstellend gelöst werden konnte.

Im Gegensatz zu körpereigenen Peptiden hat die parenterale Anwendung körperfremder Polypeptide eine grundsätzliche Schwierigkeit zu überwinden. Das Immunsystem aller höheren Organismen bildet nämlich gegen solche "non-self"-Peptide spezifische Antikörper, die versuchen, das fremde Peptid zu neutralisieren bzw. zu entfernen. Diese Antigenität fremder Peptide kann nur künstlich, z.B. mit Hilfe von Immunsuppressiva oder durch komplizierte Konstrukte unterdrückt werden, wodurch aber weitere Nebenwirkungen verursacht werden können. Als neuestes Beispiel kann Ethanercept (anti-TNF-Rezeptor-Protein) erwähnt werden, welches in der Therapie der rheumatoiden Arthritis verwendet wird.

Körperfremde Polypeptide werden im Organismus durch proteolytische Enzyme abgebaut. Die dadurch entstehenden kleineren Peptid-Segmente sind die eigentlichen Auslöser der Antigen-Reaktionen, welche zur Antikörper-Bildung führen. Polypeptide, die gegenüber proteolytischen Enzymen stabil sind, sollten im Prinzip auch eine deutlich geringere Antigenität zeigen. Substanzen, die proteolytische Enzyme wirksam inhibieren, können in der Therapie mehrerer Pathologien verwendet werden. Solche Protease-Inhibitoren wurden bereits für die Behandlung der HIV-Infektion eingesetzt.

Thionine sind kleine, stark basische Peptide pflanzlicher Herkunft, die durch einen ungewöhnlich hohen Cysteingehalt charakterisiert sind. Die Thionine sind meist aus 45 bis 48 Aminosäure-Einheiten aufgebaut (Römpp Lexikon der Chemie 9. Auflage, Thieme Verlag Stuttgart 1992). Sie enthalten meist 6 oder 8 Cystein-Gruppen, die dementsprechend drei oder vier intramolekulare Disulfid-Brücken (-S-S-) bilden und dadurch die Struktur des Peptids zusätzlich stabilisieren. Aufgrund ihres relativ kleinen (ca. 5 kDa) Molekulargewichts und ihrer außergewöhnlich stabilen Struktur ist zu erwarten, dass Thionine mehrere, pharmakologisch spezifische Eigenschaften aufweisen, die bis jetzt jedoch nur in geringem Maße erkannt und angewendet wurden.

In der Fachliteratur wurden bisher nur ca. 50 einzelne Thionine beschrieben. Die bekanntesten Thionine sind Viscotoxine aus Mistel *(Viscum album*), Hordothionine aus Gerste *(Hordeum vulgaris*), Purothionine aus Weizen *(Triticum sativum*), Avenothionine aus Hafer (*Avena-sativa*), Pyrulariathionine aus Büffelnuss (*Pyrularia-pubera*) und Crambin aus *Crambus abessinicus* (D.E.A. Florack und W.J. Stiekema, Plant. Mol. Biol. 26, 25-37 (1994)).

Die genaue physiologische Wirkung der pflanzlichen Thionine konnte bis jetzt nicht eindeutig aufgeklärt werden. Es wurde bei einigen Thionin-produzierenden Pflanzen festgestellt, dass diese bei Infektionen mit Bakterien oder Pilzen mit einer erhöhten Exprimierung ihrer Thionine reagieren. Aus diesem Grunde wurde angenommen, dass Thionine eine Art Abwehrstoff-Funktion erfüllen könnten. Folglich wurde der Einbau von Thionin-Genen aus Hafer *(Avena sativa*) in andere, Thionin-freie Nutzpflanzen mit der Absicht vorgenommen, neue Nutzpflanzensorten (z.B. Reis oder Kartoffel) mit erhöhter Resistenz gegen Fungi und Bakterien zu züchten (US 5,942,663; US 6,187,995).

Durch den Einbau der alpha-Thionin-Gensequenzen aus der Gerste in das Genom der Tabakpflanze wurde z.B. eine gegen *Psudomonas-syringae* resistente Tabak-Sorte erzeugt (M.J. Carmona, A. Molina, J.A. Lopez-Fando und F. Garcia-Olmedo, Plant J. 3, 457-462 (1993)). Die Inhibition von *Phytophora infestans* an Kartoffelblätter durch Thionine wurde ebenfalls beschrieben (A. Molina, P.A. Groy, A. Fraile, R. Sanchez-Monge und F. Garcia-Olmedo, Plant Science 92, 672-679 (1993)).

Der Einbau von Thionin produzierenden Genen öffnet den Weg für die Züchtung einer großen Vielfalt von neuen Pflanzensorten mit deutlich erhöhter Resistenz gegen Schädlinge. Dadurch könnte die Mensch und Umwelt stark belastende Verwendung von Pestiziden deutlich reduziert werden. Die essentielle Voraussetzung für diese Art der Verwendung ist neben der hohen Wirksamkeit der eingesetzten Thionine deren möglichst geringe Toxizität für Tier und Mensch. Die Wirksamkeit eines bestimmten Thionins ist meistens nur gegen bestimmte Schädlinge bzw. Pathogene effizient, sodass neue Thionin-Strukturen für den Kampf gegen weitere Pathogene gefunden werden müssen.

Thionine werden sehr oft als allgemein toxisch für Tiere, Insekten, Bakterien, Hefe- und Säugetier-Zellen bezeichnet (D.E.A. Florack und W.J. Stiekema, Plant. Mol. Biol. 26, 25-37 (1994)). Einzelne Thionine weisen jedoch eine sehr stark voneinander abweichende Toxizität auf, die sowohl von der Struktur der Thionine wie auch von dem konkret verwendeten Verabreichungsweg abhängig ist. Viscotoxin B zum Beispiel zeigt eine geringe Toxizität, obwohl seine Struktur sich nur an 4 Aminosäure-Positionen von der Sequenz des deutlich toxischeren Viscotoxins A-1 unterscheidet. Die toxische Wirkung verschiedener Thionine wird im Allgemeinen durch die Permeabilisierung der cytosolischen Membran für Alkaliionen und der dadurch verursachten Nekrose der Zellen erklärt. Dieser Wirkmechanismus auf zellulärer Ebene wurde in einigen Fällen wie z.B. der Bildung von Membranionenkanälen durch Pyrularia Thionine detailliert untersucht (Hughes P. et al J. Biol. Chem. (2000) 275, 823-827). In einer kürzlich durchgeführten Studie wurde jedoch gezeigt, dass Viscotoxine für humane Lymphozyten toxisch sind, Purothione aus Weizen aber keine toxische Wirkung auf die selben Lymphozyten ausüben (Büssing A. et al. Eur. J. Biochem. (1999) 262, 79-87).

Beachtliche Mengen von Thioninen befinden sich in den meisten zur Ernährung verwendeten Getreidesorten, weshalb sie auch in zahlreichen Futter- und Nahrungsmitteln enthalten sind. Die per-orale Toxizität dieser Thionine wurde aber meist als nicht signifikant für Menschen und Tiere gefunden, vermutlich wegen des Abbaus der Thionine im Magen-Darm-Trakt und wegen der thermischen Zersetzung im Backofen. Eine unmittelbare humantherapeutische Anwendung eines Thionins als Reinsubstanz oder eines standardisierten Gemisches von reinen Thioninen wurde in der Fachliteratur bisher nicht beschrieben.

Es ist bekannt, dass einige in der adjuvanten Krebstherapie seit längerer Zeit verwendeten Präparate aus Mistel *(Viscum album*) kleinere Mengen von Thioninen enthalten, die als Viscotoxine bezeichnet werden. Bezüglich der onkologischen Wirksamkeit dieser Viscotoxine liegen jedoch keine gesicherten Kenntnisse vor. Mehrere Autoren behaupten sogar ausdrücklich, dass die Viscotoxine für die krebstherapeutische Wirkung der Mistel-Präparate keine Relevanz besitzen (US 5,547,674).

Die handelsüblichen Mistel-Präparate sind deswegen auch nicht auf ihren Viscotoxin-Gehalt standardisiert. Ihre tatsächliche Viscotoxin-Konzentration ist variabel, liegt aber in allen Fällen unter der Grenze von 1 µg/ml. Ausgehend von der normalen Dosis von 2-4 ml Mistel-Extrakt Injektionslösung erhält ein Patient also höchstens 0,004 mg Viscotoxin pro Tag. Mit der bei Säugetieren ermittelten Toxizität der Viscotoxine von LD₅₀ = 0,1-1 mg/kg kann bei einem erwachsenen Mensch (70 kg Körpergewicht) in der Tat keine relevante zytotoxische Wirkung auftreten, die zur Abtötung von Tumorzellen führen könnte.

In der Fachliteratur wird die onkologische Wirksamkeit der Mistel-Präparate überwiegend den im Extrakt enthaltenen Lektinen (Zucker-bindende Proteine) zugeschrieben (DE 4 221 836). Diese Mistel-Lektine zeigen in geringen Dosierungen (1 ng/kg) eine immunstimulierende Wirkung, die in vitro mehrfach bestätigt wurde (EP-A-0 602 686). Mehrere handelsübliche Mistelpräparate werden demzufolge auf ihren Gehalt an Mistel-Lektinen standardisiert. Trotzdem konnte die in vivo krebstherapeutische Wirksamkeit von rein isolierten Mistel-Lektinen nicht bestätigt werden. Mehrere Autoren vermuten daher einen Beitrag von Mistel-Polysachariden oder von weiteren bisher nicht identifizierten Komponenten der Mistel-Extrakte (J.R. Ochocka und A. Piotrowski, Can. J. Plant Pathol. 24, 21-28 (2002)).

Es wurde neuerdings vorgeschlagen, die bei kleineren Konzentrationen des Pyrularia Thionins beobachtete immunstimulierende Wirkung zur Tumorbekämpfung einzusetzen (WO 02/22159). In der WO 02/22159 wird auch die Verwendung des Thionins zusammen mit dem stark stimulierend wirkenden IL-2 beschrieben. In diesem Zusammenhang ist aber zu bedenken, dass eine starke, aber unspezifische Immunstimulierung meistens auch das Wachstum der Krebszellen fördert, erfahrungsgemäß sogar noch deutlich stärker als das Wachstum von normalen Zellen.

Die Publikation von F. Garcia-Olmedo et al. "Plant defense Peptides", BIOPOLYMERS, Band 47, Seiten 479 - 491 beschreibt unter anderem Thionine als antimikrobielle Peptide mit einer kompakten Struktur, welche durch 2 bis 6 Disulfidbrücken stabilisiert wird. Die Autoren machen eingehende Ausführungen zur räumlichen Struktur von Thioninen sowie deren biologischer Eigenschaften.

Thionine, die gegen Krebszellen deutlich toxischer wirken als gegen normale Zellen wären für die Krebstherapie sehr geeignet. Solche Thionine konnten aber bisher nicht identifiziert werden. Es wurde vorgeschlagen (WO 96/41608) ein Pyrularia Thionin an bestimmte, gegen Krebszellen spezifisch gerichtete CD5+ Antikörper zu binden. Dieser Komplex sollte dann zur selektiven Bekämpfung von Krebszellen eingesetzt werden.

Krebs ist keine einheitliche Krankheit sondern ein Oberbegriff für mehr als 200 verschiedene Formen maligner Erkrankungen. Nahezu jedes Gewebe kann krebsartige Entartungen hervorrufen, manchmal sogar mehrere unterschiedliche Typen. Trotz dieser Verschiedenartigkeit entstehen alle Tumoren offenbar durch die Entartung ähnlicher grundlegender Prozesse.

Die gängigen Krebstherapien versuchen die grundlegenden Charakteristika der Erkrankung zu bekämpfen, nämlich Wachstum, Invasion und Metastasierung. Für die Krebsbehandlung sind Chemotherapeutika sehr wichtige Mittel, die neben der Operation und Bestrahlung eingesetzt werden. Bestimmte bösartige Tumoren (Hodenkrebs) können mit Zytostatika erfolgreich behandelt werden. Leider können die häufigsten bösartigen Tumoren (Krebs der Brust, Lungen, und Prostata sowie des Dickdarms) mit Hilfe der Chemotherapie nicht geheilt werden. Gelingt es nicht die Entwicklung des Tumors zu stoppen, werden die Krebszellen immer aggressiver und beginnen sich nach einer bestimmten Phase auszubreiten (Metastase) und lebenswichtige Organe bis zur Funktionsunfähigkeit zu schädigen. Dadurch führen sie zum Tod der Patienten. Ein grundsätzlicher Nachteil der nicht selektiv cytotoxisch wirkenden Krebstherapeutika liegt darin, dass sie auch zahlreiche gesunde Zellen töten. Daher muss bei ihrer Verwendung mit schwerwiegenden Nebenwirkungen gerechnet werden. Ein weiterer Nachteil der gegenwärtigen Chemotherapien besteht darin, dass die Tumorzellen im Laufe der Behandlung resistent gegen die eingesetzten Zytostatika werden. Dies erfordert die ständige Erhöhung der angewendeten Dosis, womit aber gleichzeitig eine Steigerung der toxischen Nebenwirkungen verbunden ist, oder die Verwendung eines Gemisches mehrerer Zytostatika.

AIDS ist ein komplexes Krankheitssyndrom mit chronischer Beschädigung der T4-Lymphozyten und weiterer Immunzellen, das sich in Folge einer Infektion mit den HIV-Retroviren ausbildet. Die als AIDS bezeichnete allgemeine Immunschwäche etabliert sich erst nachdem das Immunsystem den Kampf gegen die Infektion verloren hat. In der AIDS-Phase hat der Patient keine Immunabwehr gegen verschiedene, meist banale Infektionen und stirbt infolge solcher Krankheiten.

Bei der Suche nach Faktoren, die neben Polypeptiden an der Regulation der zellulären und humoralen Immunvorgänge beteiligt sind, wurde vor kurzem die neue Klasse der Kohlensuboxid-Derivate entdeckt (DE 196 00 301, EP 0874 851). Diese vom anorganischen Kohlensuboxid C₃O₂ abgeleiteten Naturstoffe zeigen unter anderem eine überraschend spezifische Interaktion mit bestimmten immunologisch wichtigen Proteinen wie Immunglobuline (Ig) oder Fc Rezeptoren. Es wurde weiterhin festgestellt dass diese Kohlensuboxid-Derivate fähig sind die pharmakologisch-toxikologische Eigenschaften der hier beschriebenen Peptiden aber auch von anderen Peptiden mit hohem Cysteingehalt wesentlich zu beeinflussen

### Darstellung der Erfindung

Aufgabe der vorliegenden Erfindung ist es, Peptide bereitzustellen, die die natürlichen Abwehrkräfte pflanzlicher, tierischer oder menschlicher Organismen gegen bakterielle, fungale, virale oder andere Pathogene unterstützen. Diese Aufgabe wird erfindungsgemäß durch die cysteinhaltigen Peptide gemäß unabhängigem Patentanspruch 1, durch die Nukleinsäuresequenz gemäß unabhängigem Patentanspruch 4, durch den DNA-Vektor gemäß unabhängigem Patentanspruch 8, durch die monoklonalen Antikörper gemäß unabhängigem Patentanspruch 9, durch die pharmazeutische Zubereitung gemäß unabhängigem Patentanspruch 15 und durch die Verfahren gemäß unabhängigen Patentansprüchen 18, 20 und 22 gelöst. Weitere vorteilhafte Aspekte, Details und Ausgestaltungen der Erfindung ergeben sich aus den abhängigen Ansprüchen, der Beschreibung und der Zeichnung.

Der Aufbau der erfindungsgemäßen Peptide wird nachfolgend im Einbuchstabencode der Aminosäuren dargestellt. Es bedeutet: A: Alanin, C: Cystein, D: Asparaginsäure, E: Glutaminsäure, F: Phenylalanin, G: Glycin, H: Histidin, I: Isoleucin, K: Lysin, L: Leucin, M: Methionin, N: Asparagin, P: Prolin, Q: Glutamin, R: Arginin, S: Serin, T: Threonin, V: Valin, W: Tryptophan und Y: Tyrosin.

Die Sekundär-, Tertiär- und Quartärstruktur von Peptiden wird maßgeblich durch die Art der Seitenketten der Aminosäuren bestimmt, aus denen das jeweilige Peptid aufgebaut ist. In diesem Zusammenhang können die natürlich vorkommenden Aminosäuren in Gruppen eingeteilt werden, wobei die Art der Seitenkette das Klassifizierungskriterium darstellt. Aminosäuren mit aliphatischen Seitenketten sind Glycin, Alanin, Valin, Leucin und Isoleucin. Aminosäuren mit aliphatischen Seitenketten mit Hydroxylgruppe sind Serin und Threonin. Aminosäuren mit aromatischen Seitenketten sind Phenylalanin, Tyrosin und Tryptophan. Aminosäuren mit basischen Seitenketten sind Lysin, Arginin und Histidin. Aminosäuren mit sauren Seitenketten sind Aspartat und Glutamat. Aminosäuren mit Amidseitenketten sind Asparagin und Glutamin. Aminosäuren mit schwefelhaltigen Seitenketten sind Cystein und Methionin. Die Aminosäure Prolin besitzt eine sekundäre Aminogurppe und kann in keine der genannten Klassen eingeteilt werden. Eine besondere Bedeutung für die Beeinflussung der dreidimensionalen Struktur und daher auch eine Beeinflussung der biologischen Wirksamkeit kann insbesondere durch die Bildung der Ester-Derivate der Hydroxy Gruppen enthaltenen Aminosäuren erfolgen. Die Ester-Bildung kann dabei bevorzugt mit Phosphorsäure vorgenommen werden. Eine solche Derivatisierung kann chemisch-synthetisch oder enzymatisch mit Hilfe sogenannter Phospho-Kinasen durchgeführt.

Von Interesse sind cysteinhaltige Peptide der Struktur XXCCXXXXXXXCCXXXCXXXXXXQXXCXXXCXCXXXXXXXCCXXXXXX, der Struktur XXCCXXXXXXXCXXXCXXXXXXXXXCXXXCXCXXXXTXXCXXXXXX und der Struktur XXCCXXXXXXXCXXXCXXXXXXXXXXCXXXCXCXXXXXXXXCXXXXXX, wobei X einen Platzhalter darstellt. Sämtliche X können unabhängig voneinander gewählt werden und bedeuten jede beliebige der oben genannten, natürlich vorkommenden Aminosäuren.

Zu den natürlich vorkommenden Aminosäuren zählen neben den gängigen L-Aminosäuren auch D-Aminosäuren sowie Aminosäurederivate wie beispielsweise 4-Hydroxyprolin, N,N,N-Trimethyllysin, 3-Methylhistidin, 5-Hydroxylysin, O-Phosphoserin, γ-Carboxyglutamat, ε-N-Acetyllysin, ω-N-Methylarginin, Citrullin oder Omithin.

Bevorzugt werden die Disulfidbrücken zwischen den im folgenden gezeigten Cysteinen ausgebildet, so dass sich folgende bevorzugte Strukturen ergeben:

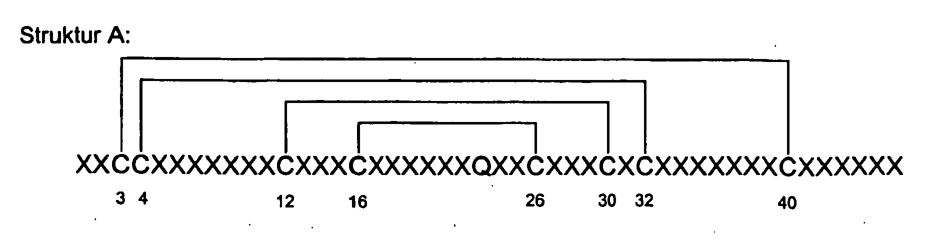

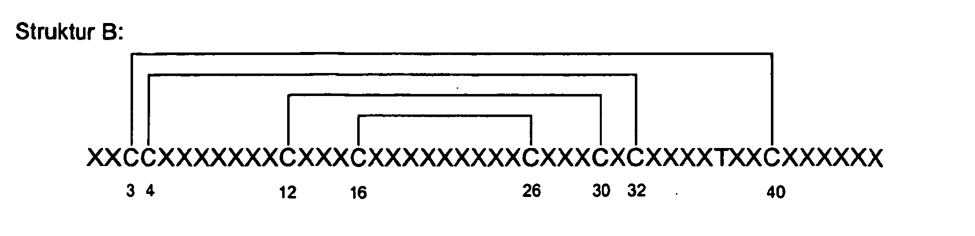

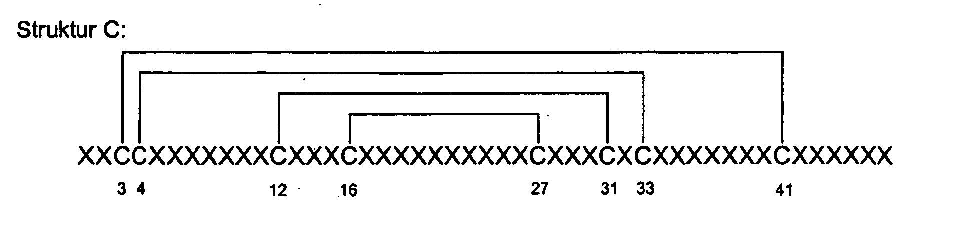

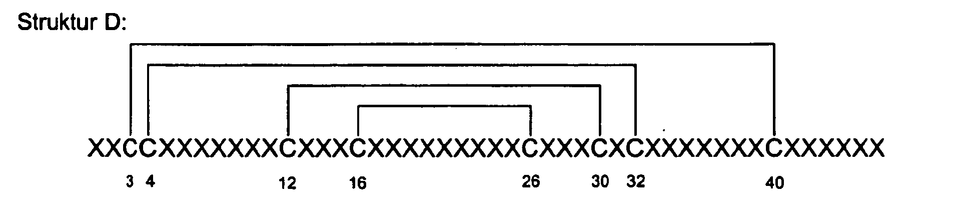

Die vorliegende Erfindung betrifft cysteinhaltige Peptide der folgenden Struktur: 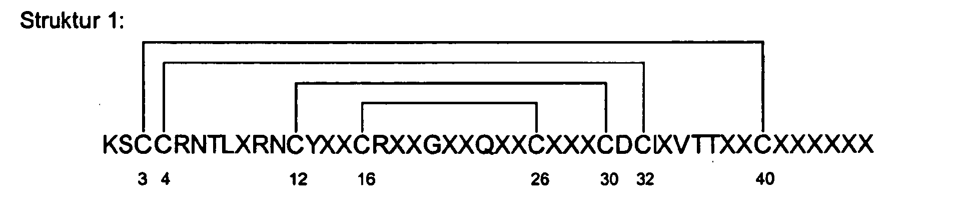 oder 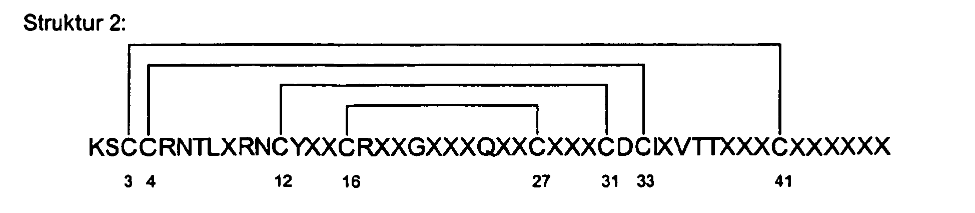 welche die natürlichen Abwehrkräfte pflanzlicher, tierischer oder menschlicher Organismen gegen bakterielle, fungale, virale oder andere Pathogene unterstützen, wobei die durchgezogenen Linien die Disulfidbrücken symbolisieren und wobei X unabhängig voneinander jede beliebige natürlich vorkommende Aminosäure darstellt.

Ferner sind folgende Peptide der Struktur 1 bevorzugt, worin sich an Position 15 die Aminosäure G und/oder an Position 19 die Aminosäure T und/oder an Position 27 die Aminosäure Q und/oder an Position 28 die Aminosäure R und/oder an Position 34 die Aminosäure H und/oder an Position 38 die Aminosäure T und/oder an Position 43 die Aminosäure S und/oder an Position 44 die Aminosäure H und/oder an Position 46 die Aminosäure S befindet.

Ferner sind folgende Peptide der Struktur 2 bevorzugt, worin sich die folgenden Aminosäurekombinationen befinden:

| Position | AS | | Position | AS | | Position | AS | |
|---|---|---|---|---|---|---|---|---|
| 15 | G | und | 19 | T | oder | 23 | Q | oder |
| | | | 28 | G | oder | 29 | R | oder |
| | | | 32 | D | oder | 34 | I | oder |
| | | | 35 | H | oder | 36 | V | oder |
| | | | 37 | T | oder | 38 | T | oder |
| | | | 39 | T | oder | 44 | S | oder |
| | | | 45 | H | oder | 47 | S | |
| | | | | | | | | |
| 19 | T | und | 15 | G | oder | 23 | Q | oder |
| | | | 28 | G | oder | 29 | R | oder |
| | | | 32 | D | oder | 34 | I | oder |
| | | | 35 | H | oder | 36 | V | oder |
| | | | 37 | T | oder | 38 | T | oder |
| | | | 39 | T | oder | 44 | S | oder |
| | | | 45 | H | oder | 47 | S | |
| | | | | | | | | |
| 23 | Q | und | 15 | G | oder | 19 | T | oder |
| | | | 28 | G | oder | 29 | R | oder |
| | | | 32 | D | oder | 34 | I | oder |
| | | | 35 | H | oder | 36 | V | oder |
| | | | 37 | T | oder | 38 | T | oder |
| | | | 39 | T | oder | 44 | S | oder |
| | | | 45 | H | oder | 47 | S | |
| | | | | | | | | |
| 24 | Q | und | 15 | G | oder | 19 | T | oder |
| | | | 28 | G | oder | 29 | R | oder |
| | | | 32 | D | oder | 34 | I | oder |
| | | | 35 | H | oder | 36 | V | oder |
| | | | 37 | T | oder | 38 | T | oder |
| | | | 39 | T | oder | 44 | S | oder |
| | | | 45 | H | oder | 47 | S | |
| | | | | | | | | |
| 28 | Q | und | 15 | G | oder | 19 | T | oder |
| | | | 23 | Q | oder | 29 | R | oder |
| | | | 32 | D | oder | 34 | I | oder |
| | | | 35 | H | oder | 36 | V | oder |
| | | | 37 | T | oder | 38 | T | oder |
| | | | 39 | T | oder | 44 | S | oder |
| | | | 45 | H | oder | 47 | S | |
| | | | | | | | | |
| 29 | R | und | 15 | G | oder | 19 | T | oder |
| | | | 23 | Q | oder | 29 | R | oder |
| | | | 32 | D | oder | 34 | I | oder |
| | | | 35 | H | oder | 36 | V | oder |
| | | | 37 | T | oder | 38 | T | oder |
| | | | 39 | T | oder | 44 | S | oder |
| | | | 45 | H | oder | 47 | S | |
| | | | | | | | | |
| 32 | D | und | 15 | G | oder | 19 | T | oder |
| | | | 23 | Q | oder | 28 | Q | oder |
| | | | 29 | R | oder | 34 | I | oder |
| | | | 35 | H | oder | 36 | V | oder |
| | | | 37 | T | oder | 38 | T | oder |
| | | | 39 | T | oder | 44 | S | oder |
| | | | 45 | H | oder | 47 | S | |
| | | | | | | | | |
| 34 | I | und | 15 | G | oder | 19 | T | oder |
| | | | 23 | Q | oder | 28 | Q | oder |
| | | | 29 | R | oder | 32 | D | oder |
| | | | 35 | H | oder | 36 | V | oder |
| | | | 37 | T | oder | 38 | T | oder |
| | | | 39 | T | oder | 44 | S | oder |
| | | | 45 | H | oder | 47 | S | |
| | | | | | | | | |
| 35 | H | und | 15 | G | oder | 19 | T | oder |
| | | | 23 | Q | oder | 28 | Q | oder |
| | | | 29 | R | oder | 32 | D | oder |
| | | | 34 | I | oder | 36 | V | oder |
| | | | 37 | T | oder | 38 | T | oder |
| | | | 39 | T | oder | 44 | S | oder |
| | | | 45 | H | oder | 47 | S | |
| | | | | | | | | |
| 36 | V | und | 15 | G | oder | 19 | T | oder |
| | | | 23 | Q | oder | 28 | Q | oder |
| | | | 29 | R | oder | 32 | D | oder |
| | | | 34 | I | oder | 35 | H | oder |
| | | | 37 | T | oder | 38 | T | oder |
| | | | 39 | T | oder | 44 | S | oder |
| | | | 45 | H | oder | 47 | S | |
| | | | | | | | | |
| 37 | T | und | 15 | G | oder | 19 | T | oder |
| | | | 23 | Q | oder | 28 | Q | oder |
| | | | 29 | R | oder | 32 | D | oder |
| | | | 34 | I | oder | 35 | H | oder |
| | | | 36 | V | oder | 38 | T | oder |
| | | | 39 | T | oder | 44 | S | oder |
| | | | 45 | H | oder | 47 | S | |
| | | | | | | | | |
| 38 | T | und | 15 | G | oder | 19 | T | oder |
| | | | 23 | Q | oder | 28 | Q | oder |
| | | | 29 | R | oder | 32 | D | oder |
| | | | 34 | I | oder | 35 | H | oder |
| | | | 36 | V | oder | 37 | T | oder |
| | | | 39 | T | oder | 44 | S | oder |
| | | | 45 | H | oder | 47 | S | |
| | | | | | | | | |
| 39 | T | und | 15 | G | oder | 19 | T | oder |
| | | | 23 | Q | oder | 28 | Q | oder |
| | | | 29 | R | oder | 32 | D | oder |
| | | | 34 | I | oder | 35 | H | oder |
| | | | 36 | V | oder | 37 | T | oder |
| | | | 38 | T | oder | 44 | S | oder |
| | | | 45 | H | oder | 47 | S | |
| | | | | | | | | |
| 44 | S | und | 15 | G | oder | 19 | T | oder |
| | | | 23 | Q | oder | 28 | Q | oder |
| | | | 29 | R | oder | 32 | D | oder |
| | | | 34 | I | oder | 35 | H | oder |
| | | | 36 | V | oder | 37 | T | oder |
| | | | 38 | T | oder | 39 | T | oder |
| | | | 45 | H | oder | 47 | S | |
| | | | | | | | | |
| 45 | H | und | 15 | G | oder | 19 | T | oder |
| | | | 23 | Q | oder | 28 | Q | oder |
| | | | 29 | R | oder | 32 | D | oder |
| | | | 34 | I | oder | 35 | H | oder |
| | | | 36 | V | oder | 37 | T | oder |
| | | | 38 | T | oder | 39 | T | oder |
| | | | 44 | S | oder | 47 | S | |
| | | | | | | | | |
| 47 | S | und | 15 | G | oder | 19 | T | oder |
| | | | 23 | Q | oder | 28 | Q | oder |
| | | | 29 | R | oder | 32 | D | oder |
| | | | 34 | I | oder | 35 | H | oder |
| | | | 36 | V | oder | 37 | T | oder |
| | | | 38 | T | oder | 39 | T | oder |
| | | | 44 | S | oder | 45 | H | |

Ferner sind folgende Peptide der Struktur 1 bevorzugt, worin sich die folgenden Aminosäurekombinationen befinden:

| Position | AS | | Position | AS | | Position | AS | |
|---|---|---|---|---|---|---|---|---|
| 15 | G | und | 19 | T | oder | 27 | Q | oder |
| | | | 28 | R | oder | 31 | D | oder |
| | | | 33 | I | oder | 34 | H | oder |
| | | | 35 | V | oder | 36 | T | oder |
| | | | 37 | T | oder | 38 | T | oder |
| | | | 43 | S | oder | 44 | H | oder |
| | | | 46 | S | | | | |
| | | | | | | | | |
| 19 | T | und | 15 | G | oder | 27 | Q | oder |
| | | | 28 | R | oder | 31 | D | oder |
| | | | 33 | I | oder | 34 | H | oder |
| | | | 35 | V | oder | 36 | T | oder |
| | | | 37 | T | oder | 38 | T | oder |
| | | | 43 | S | oder | 44 | H | oder |
| | | | 46 | S | | | | |
| | | | | | | | | |
| 27 | Q | und | 15 | G | oder | 19 | T | oder |
| | | | 28 | R | oder | 31 | D | oder |
| | | | 33 | I | oder | 34 | H | oder |
| | | | 35 | V | oder | 36 | T | oder |
| | | | 37 | T | oder | 38 | T | oder |
| | | | 43 | S | oder | 44 | H | oder |
| | | | 46 | S | | | | |
| | | | | | | | | |
| 28 | R | und | 15 | G | oder | 19 | T | oder |
| | | | 27 | Q | oder | 31 | D | oder |
| | | | 33 | I | oder | 34 | H | oder |
| | | | 35 | V | oder | 36 | T | oder |
| | | | 37 | T | oder | 38 | T | oder |
| | | | 43 | S | oder | 44 | H | oder |
| | | | 46 | S | | | | |
| | | | | | | | | |
| 31 | D | und | 15 | G | oder | 19 | T | oder |
| | | | 27 | Q | oder | 28 | R | oder |
| | | | 33 | I | oder | 34 | H | oder |
| | | | 35 | V | oder | 36 | T | oder |
| | | | 37 | T | oder | 38 | T | oder |
| | | | 43 | S | oder | 44 | H | oder |
| | | | 46 | S | | | | |
| | | | | | | | | |
| 33 | I | und | 15 | G | oder | 19 | T | oder |
| | | | 27 | Q | oder | 28 | R | oder |
| | | | 31 | D | oder | 34 | H | oder |
| | | | 35 | V | oder | 36 | T | oder |
| | | | 37 | T | oder | 38 | T | oder |
| | | | 43 | S | oder | 44 | H | oder |
| | | | 46 | S | | | | |
| | | | | | | | | |
| 34 | H | und | 15 | G | oder | 19 | T | oder |
| | | | 27 | Q | oder | 28 | R | oder |
| | | | 31 | D | oder | 33 | I | oder |
| | | | 35 | V | oder | 36 | T | oder |
| | | | 37 | T | oder | 38 | T | oder |
| | | | 43 | S | oder | 44 | H | oder |
| | | | 46 | S | | | | |
| | | | | | | | | |
| 35 | V | und | 15 | G | oder | 19 | T | oder |
| | | | 27 | Q | oder | 28 | R | oder |
| | | | 31 | D | oder | 33 | I | oder |
| | | | 34 | H | oder | 36 | T | oder |
| | | | 37 | T | oder | 38 | T | oder |
| | | | 43 | S | oder | 44 | H | oder |
| | | | 46 | S | | | | |
| | | | | | | | | |
| 36 | T | und | 15 | G | oder | 19 | T | oder |
| | | | 27 | Q | oder | 28 | R | oder |
| | | | 31 | D | oder | 33 | I | oder |
| | | | 34 | H | oder | 35 | V | oder |
| | | | 37 | T | oder | 38 | T | oder |
| | | | 43 | S | oder | 44 | H | oder |
| | | | 46 | S | | | | |
| | | | | | | | | |
| 38 | T | und | 15 | G | oder | 19 | T | oder |
| | | | 27 | Q | oder | 28 | R | oder |
| | | | 31 | D | oder | 33 | I | oder |
| | | | 34 | H | oder | 35 | V | oder |
| | | | 36 | T | oder | 37 | T | oder |
| | | | 43 | S | oder | 44 | H | oder |
| | | | 46 | S | | | | |
| | | | | | | | | |
| 43 | S | und | 15 | G | oder | 19 | T | oder |
| | | | 27 | Q | oder | 28 | R | oder |
| | | | 31 | D | oder | 33 | I | oder |
| | | | 34 | H | oder | 35 | V | oder |
| | | | 36 | T | oder | 37 | T | oder |
| | | | 38 | T | oder | 44 | H | oder |
| | | | 46 | S | | | | |
| | | | | | | | | |
| 44 | H | und | 15 | G | oder | 19 | T | oder |
| | | | 27 | Q | oder | 28 | R | oder |
| | | | 31 | D | oder | 33 | I | oder |
| | | | 34 | H | oder | 35 | V | oder |
| | | | 36 | T | oder | 37 | T | oder |
| | | | 38 | T | oder | 43 | S | oder |
| | | | 46 | S | | | | |
| | | | | | | | | |
| 46 | S | und | 15 | G | oder | 19 | T | oder |
| | | | 27 | Q | oder | 28 | R | oder |
| | | | 31 | D | oder | 33 | I | oder |
| | | | 34 | H | oder | 35 | V | oder |
| | | | 36 | T | oder | 37 | T | oder |
| | | | 38 | T | oder | 43 | S | oder |
| | | | 44 | H | | | | |

Die Abkürzung AS steht für Aminosäure.

Ganz besonders bevorzugt werden cysteinhaltige Peptide der Struktur KSCCRNTLGRNCYNGCRFTGGSQPTCGRLCDCIHVTTTTCPSSHPS (Hellethionin-A), KSCCRNTLGRNCYNACRFTGGSQPTCGRLCDCIHVTTTTCPSSHPS (Hellethionin-B1), KSCCRNTLARNCYNACRFTGGSQPTCGRLCDCIHVTTTTCPSSHPS (Hellethionin-B2), KSCCRNTLGRNCYNACRLPGTPQPTCATLCDCIHVTTPTCPSSHPR (Hellethionin-B3), KSCCRNTLARNCYNACRFTGTSQPYCARLCDCIHVTTPTCPSSHPR (Hellethionin-B4), KSCCRNTLARNCYNACRFTGGSQPTCATLCDCIHVTTPTCPSSHPR (Hellethionin-B5), KSCCRNTLARNCYNVCRFGGGSQAYCARFCDCIHVTTSTCPSSHPS (Hellethionin-B6), KSCCRNTLGRNCYNACRLTGTSQATCATLCDCIHVTATTCRPPYPS (Hellethionin-C), KSCCRNTLARNCYNACRFTGGSQPTCGILCDCIHVTTTTCPSSHPS (Hellethionin-D), KSCCRNTLGRNCYAACRLTGLFSQEQCARLCDCITVTTPTPCPRTHPS (Hellethionin-E1) und KSCCRNTLGRNCYAACRLTGTFSQEQCARLCDCITVTTPTPCPRTHPS (Hellethionin-E2).

Ferner sind insbesondere noch Derivate der vorgenannten Hellethionine, d.h. Derivate von Hellethionin-A, Hellethionin-B1, Hellethionin-B2, Hellethionin-B3, Hellethionin-B4, Hellethionin-B5, Hellethionin-B6, Hellethionin-C, Hellethionin-D und Hellethionin-E1 bevorzugt, worin die folgenden Aminosäuren (ursprüngliche AS) durch die nebenstehenden Aminosäuren (Austausch-AS) ersetzt worden sind:

| ursprüngliche AS | Austausch-AS |
|---|---|
| Ala | Ser |
| Arg | Lys |
| Asn | GIn oder His |
| Asp | Glu |
| Cys | Ser |
| Gin | Asn |
| Glu | Asp |
| Gly | Pro |
| His | Asn oder Gln |
| Ile | Leu oder Val |
| Leu | Ile oder Val |
| Lys | Arg oder Gln oder Glu |
| Met | Leu oder Ile |
| Phe | Met oder Leu oder Tyr |
| Ser | Thr |
| Thr | Ser |
| Trp | Tyr |
| Tyr | Trp oder Phe |
| Val | Ile oder Leu |

Ferner ist auch die Verwendung von Peptidomimetika möglich. Hierbei handelt es sich um chemische Substanzen, welche eines der vorgenannten Peptide imitiert.

Von der vorliegenden Erfindung umfasst sind auch Ester-Derivate, Amid-Derivate, Salz-Derivate, cyclische Derivate und Derivate mit einem modifizierten Rückgrat der genannten Peptide.

Aminosäuren mit Carboxylatgruppen können beispielsweise in ein Salz überführt werden, oder in einen Ester, bevorzugt einen C₁-C₁₆-Ester oder ein Amid, optional mit einem oder zwei Alkylresten, bevorzugt mit C₁-C₁₆-Alkylresten. Hydroxylgruppen, beispielsweise des Tyrosins oder Serins können in einen Ester oder Ether überführt werden. Ferner ist auch die Bildung von Acetalen, Ketalen oder Carbonaten möglich. Derartige Reste bestehen bevorzugt aus 1 bis 16 Kohlenstoffatomen. Ferner können auch alle anderen bekannten OH-Schutzgruppen eingesetzt werden. Die bei den Schutzgruppen verwendeten Alkylrest können weitere Substituenten tragen, beispielsweise Halogene, Aminogruppen, Hydroxygruppen, Carbonylgruppen, Thiolgruppen, Arylgruppen, Aklylverzeigungen, Carboxylgruppen, Nitrogruppen, Amidgruppen und/oder Estergruppen.

Ganz besonders bevorzugt im Rahmen der vorliegenden Erfindung werden die cysteinhaltigen Peptidverbindungen Hellethionin-A, Hellethionin-B1, Hellethionin-B2, Hellethionin-B3, Hellethionin-B4, Hellethionin-B5, Hellethionin-B6, Hellethionin-C, Hellethionin-D, Hellethionin-E1 und Hellethionin-E2 sowie Derivate dieser cysteinhaltigen Peptidverbindungen mit Aminosäure-Variationen an bis zu 15 Positionen der cysteinhaltigen Peptidverbindung bzw. an bis zu 15 Positionen der Derivate dieser cysteinhaltigen Peptidverbindungen, wobei die Aminosäure Cystein bezüglich Position und Art keiner Variation unterworfen ist. Insbesondere bevorzugt ist es, wenn die Aminosäure-Variationen an bis zu 13 Positionen, an bis zu 11 Positionen, an bis zu 9 Positionen, an bis zu 7 Positionen, an bis zu 5 Positionen, an bis zu 4 Positionen, an bis zu 3 Positionen, an bis zu 2 Positionen bzw. nur an einer Position der Peptidverbindungen bzw. der Derivate der Peptidverbindungen auftreten.

Insbesondere bevorzugt ist es, wenn die cysteinhaltigen Peptidverbindungen Hellethionin-A, Hellethionin-B1, Hellethionin-B2, Hellethionin-B3, Hellethionin-B4, Hellethionin-B5, Hellethionin-B6, Hellethionin-C, Hellethionin-D, Hellethionin-E1 und Hellethionin-E2 sowie Derivate dieser cysteinhaltigen Peptidverbindungen eine der oben genannten Anzahlen an Aminosäure-Variationen aufweisen und sich gleichzeitig an Position 1 die Aminosäure K und/oder an Position 2 die Aminosäure S und/oder an Position 5 die Aminosäure R und/oder an Position 6 die Aminosäure N und/oder an Position 7 die Aminosäure T und/oder an Position 8 die Aminosäure L und/oder an Position 10 die Aminosäure R und/oder an Position 11 die Aminosäure N und/oder an Position 13 die Aminosäure Y und/oder an Position 17 die Aminosäure R und/oder an Position 20 die Aminosäure G befindet.

Besonders bevorzugte Ausführungsformen der vorliegenden Erfindung beziehen sich auf cysteinhaltige Peptide der genannten allgemeinen Strukturen, wobei sich an bestimmten Positionen des Peptids Aminosäuren befinden, die aus einer bestimmten der oben definierten Gruppen von Aminosäuren ausgewählt sind. Bevorzugt werden Peptide, wobei sich an Position 1 eine Aminosäure mit basischer Seitenkette und/oder an Position 2 eine Aminosäure mit aliphatischer Seitenkette mit Hydroxylgruppe und/oder an Position 5 eine Aminosäure mit basischer Seitenkette und/oder an Position 6 eine Aminosäure mit Amidseitenkette und/oder an Position 7 eine Aminosäure mit aliphatischer Seitenkette mit Hydroxylgruppe und/oder an Position 8 eine Aminosäure mit aliphatischer Seitenkette und/oder an Position 10 eine Aminosäure mit basischer Seitenkette und/oder an Position 11 eine Aminosäure mit Amidseitenkette und/oder an Position 13 eine Aminosäure mit aromatischer Seitenkette und/oder an Position 17 eine Aminosäure mit basischer Seitenkette und/oder an Position 20 eine Aminosäure mit aliphatischer Seitenkette befindet.

Die vorliegende Erfindung umfasst selbstverständlich auch Peptide der genannten allgemeinen Strukturen, die an einem oder beiden ihrer Enden funktionale Modifikationen tragen. Insbesondere sind auch längerkettige Peptide umfasst, also Peptide, deren Aminosäure-Kette über die genannten allgemeinen Strukturen hinausreicht. Die erfindungsgemäßen Peptide stellen in diesen Fällen also nur einen Abschnitt eines größeren Peptids dar. Die Funktionalität der erfindungsgemäßen Peptide, also deren Wirkung, darf durch diese funktionalen Modifikationen nicht signifikant beeinträchtigt werden.

Von den erfindungsgemäßen Strukturen 1 sowie 2 sind auch Peptide umfasst, welche vor dem K an Position 1 und/oder dem X an Position 46 bzw. 47 beliebige Endgruppen tragen oder noch einen weitere Oligopeptidkette mit bis zu 50 Aminosäuren, bevorzugt mit bis zu 30 Aminosäuren, weiter bevorzugt mit bis zu 15 Aminosäuren und insbesondere bevorzugt mit bis zu 7 Aminosäuren aufweisen.

Daneben bezieht sich die vorliegende Erfindung auf die Nukleinsäuresequenzen, die die genannten cysteinhaltigen Peptide codieren, und ebenso auf die entsprechenden RNA-Sequenzen und die entsprechenden DNA-Sequenzen sowie die entsprechende Anti-Sense DNA und die entsprechende Anti-Sense RNA. Von der vorliegenden Erfindung werden auch DNA-Vektoren und DNA-Konstrukte umfasst, die eine einem erfindungsgemäßen Peptid entsprechende cDNA oder DNA sowie einen passenden Promotor und gegebenenfalls einen passenden Verstärker (enhancer) enthalten.

Daneben sind auch monoklonale Antikörper umfasst, die gegen ein Epitop der genannten cysteinhaltigen Peptide gerichtet sind.

Die erfindungsgemäßen Peptide mit hohem Cysteingehalt sind aus 46 bzw. 48 Aminosäuren aufgebaut. Die Zählung der Positionen erfolgt ausgehend vom freien NH₂-Ende der Aminosäure-Kette.

Von den oben genannten Hellethioninen wurden die mit Hellethionin-A (HT-A), Hellethionin-C (HT-C) und die mit Hellethionin-D (HT-D) bezeichneten Peptide durch die nachfolgend beschriebenen Methoden einzeln isoliert. Im Falle von Hellethionin-B und Hellethionin-E liegt ein Gemisch mehrerer Isoformen vor, wobei die Aminosäuresequenz von sechs Isoformen (HT-B1, HT-B2, HT-B3, HT-B4, HT-B5, HT-B6) bzw. von zwei Isoformen (HT-E1 und HT-E2) aufgeklärt wurde. Die erfindungsgemäßen Peptide können als Reinsubstanzen, als Gemisch von Isoformen oder als Gemisch von Isoformen zusammen mit einem oder mehreren anderen Hellethioninen, bevorzugt als Gemisch von mehreren Hellethioninen verwendet werden.

Die Aminosäuresequenz der isolierten oder synthetisch hergestellten erfindungsgemäßen Peptide wurde durch sukzessiven Abbau der Peptidkette nach der Edman Methode und nachfolgender HPLC-Identifizierung der PTH (Phenylthiohydanthoin)-Derivate bestimmt. Die bei dieser Methode übliche enzymatische Fragmentierung der Peptide konnte wegen ihrer erhöhten enzymatischen Stabilität nicht unmittelbar durchgeführt werden. Aus diesem Grunde war zuerst eine mit Hilfe von Vinylpyridin durchgeführte Reduktion bzw. Öffnung der Disulfid-Brücken erforderlich.

Eine weitere Bestätigung der Aminosäure-Zusammensetzung der erfindungsgemäßen Peptide erfolgte durch die Bestimmung ihrer molaren Masse. Zu diesem Zweck wurde die Massenspektrometrie mit ESI (electro-spray-ionization) und die MALDI (matrix-assistedlaser-desorption-ionization) Technik angewendet.

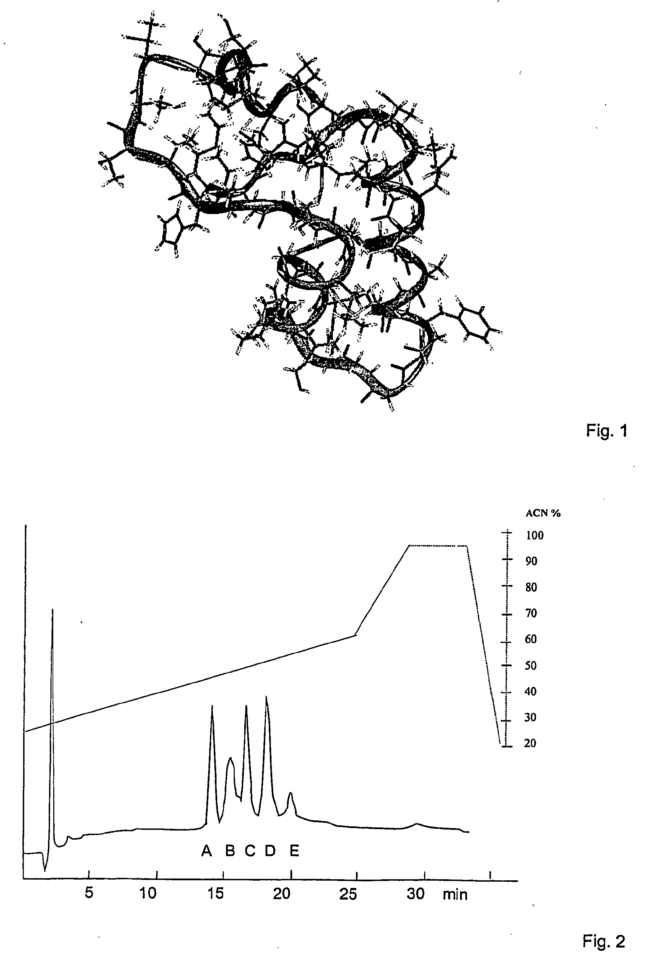

Die durch NMR-Spektroskopie bestimmte dreidimensionale Struktur des Peptids HT-D ist in der Figur 1 dargestellt. Die Gesamtstruktur ähnelt der Gestalt eines großen griechischem Gamma (┌). Der lange Arm in dieser Struktur-Analogie wird durch die zwei entgegengesetzt laufenden Helices gebildet, wohingegen der kurze Arm aus dem kurzen Beta-Faltblatt besteht. Die beiden Helices sind durch eine Schleife zwischen den Aminosäuren in den Positionen 17 und 24 verbunden.

Ein besonderer Beitrag zur Stabilität der erfindungsgemäßen Peptide wird durch die insgesamt vier Disulfid-Brücken zwischen den Cystein-Einheiten in den Positionen 3 - 40 (bzw. 42), 4 - 32, 12 - 30 (bzw. 31) und 16 - 26 (bzw. 27) gewährleistet. Die dadurch erreichte außergewöhnliche Stabilität gegenüber proteolytischen und anderen Enzymen ist eine spezifische Eigenschaft mit wichtiger Bedeutung für die Anwendung der hier beschriebenen Peptide.

Die im Rahmen der vorliegenden Erfindung bevorzugten Peptide sind durch eine erstaunlich hohe Konstanz ihrer Aminosäure-Sequenzen gekennzeichnet. Diese Konstanz betrifft bis zu 36 Positionen, also ungefähr ¾ der Gesamtkette. Die Variationen in der Zusammensetzung der Polypeptidkette ist meist auf 15 oder noch weniger Stellen begrenzt.

Insbesondere konstant ist die Lage der Cystein-Reste, die sich in den erfindungsgemäßen Peptiden an den Positionen 3, 4, 12, 16, 26 bzw. 27, 30 bzw. 31, 32 und 40 bzw. 42 befinden. In den mit HT-E1 und HT-E2 bezeichneten Sequenzen sind die letzten drei Cystein-Einheiten um je eine (27 statt 26) (31 statt 30) bzw. um zwei Positionen (42 statt 40) verschoben. Die Cystein-Einheiten werden paarweise durch Disulfid-Brücken verbunden. Aus den zahlreichen möglichen Kombinationen von Cystein-Einheiten werden in den erfindungsgemäßen Peptiden die Verknüpfungen 3 → 40 (bzw. 42), 4 → 32, 12 → 30 (bzw. 31) und 16 → 26 (bzw. 27) bevorzugt ausgebildet, wodurch eine bestimmte Sekundärstruktur der erfindungsgemäßen Peptide definiert wird.

Die Verwendung der erfindungsgemäßen Peptide bringt im Vergleich zu den bisher bekannten Thioninen mit 3 Disulfid-Brücken mehrere Vorteile. Erstens haben die erfindungsgemäßen Peptide durch das Vorhandensein der zusätzlichen Cystein-Brücke eine bessere Stabilität gegenüber proteolytischen Enzymen und zeigen dementsprechend eine geringere Immunogenität als z.B. die bereits erwähnten Viscotoxine. Ein weiterer Vorteil der erfindungsgemäßen Peptide liegt in ihrer erstaunlich guten Wasserlöslichkeit und der dadurch erzielten besseren Bioverfügbarkeit. Dieser Vorteil wird besonders im Vergleich zu bisher bekannten Thioninen mit 4 Disulfid-Brücken deutlich. Die früher beschriebenen Thionin-Peptide mit 4 Disulfid-Brücken wie z.B. Purothionine, Avenothionine oder Hordothionine sind stark basisch und sehr lipophil und deswegen nur gering wasserlöslich. Sie werden von dem entsprechenden Getreide-Samen mit Hilfe apolarer Lösungsmittel wie Petroläther extrahiert. Deshalb können wässrige Lösungen dieser Peptide nur schwierig hergestellt bzw. verwendet werden.

### Isolierung und Herstellung der erfindungsgemäßen Peptide

Von der vorliegenden Erfindung werden auch Verfahren zur Gewinnung der erfindungsgemäßen cysteinhaltigen Peptide umfasst. Ein solches erfindungsgemäßes Verfahren stellt die Extraktion aus der Pflanze Helleborus species dar. Besonders bevorzugt wird in diesem Fall eine als erster Verfahrensschritt durchgeführte Entfettung des Pflanzenmaterials unter Verwendung von nicht-polaren Lösungsmitteln oder Gemischen nicht-polarer Lösungsmittel, insbesondere unter Verwendung von tert.-Butylmethylether. Die Isolierung von erfindungsgemäßen Peptide mit hohem Cysteingehalt oder von Gemischen der erfindungsgemäßen Peptide erfolgt aus Pflanzen der Familie Ranunculaceae (Hahnenfußgewächse) und bevorzugt aus Pflanzen der Gattung Helleborus (Christrose). Zur Isolierung wird an Luft getrocknetes Pflanzenmaterial, bevorzugt die unterirdische Teile der Pflanze (Wurzel und Wurzelstock) verwendet. Bevorzugt wird zunächst eine Entfettung des Pflanzenmaterials mit einem nicht polaren Lösungsmittel, bevorzugt TBM (tert-Butylmethylether), durchgeführt. Die entfetteten und an Luft getrockneten Wurzeln werden anschließend mit organischem Alkohol / Säure-Gemischen, bevorzugt Methanol / Ameisensäure, oder mit Wasser enthaltenden Lösungsmittelgemischen, bevorzugt Wasser / Ethanol, bzw. mit verdünnten Säuren, bevorzugt Essigsäure 0,1-12 %, extrahiert.

Die filtrierte Extraktionslösung wird im Vakuum bis auf 1/10 des ursprünglichen Volumens eingeengt und danach mit einem Adsorptionsmaterial, bevorzugt Aktivkohle, behandelt. Das Filtrat wird im Vakuum eingeengt und der Trockenrückstand in einer minimalen Menge Wasser gelöst. Durch Zugabe von verdünnter Salzsäure wird der pH-Wert der wässrigen Lösung im sauren Bereich, bevorzugt pH 0,1 - 3,0 eingestellt. Die wässrige Lösung wird mit einem mehrfachen, bevorzugt zehnfachen Volumen eines gekühlten organischen Lösungsmittels oder Lösungsmittelgemisches, bevorzugt Ethanol / Aceton 1:3 vereinigt. Der sich bildende hell-gelbe Niederschlag wird filtriert, im Vakuum getrocknet und für das weitere Reinigungsverfahren verwendet.

Ein alternatives Verfahren zur Gewinnung der erfindungsgemäßen Peptide erfolgt durch deren selektive Anbindung an lonenaustauscherharze, bevorzugt an schwach saure Ionenaustauscher. Die am Harz gebundenen Peptide werden mit Hilfe einer Behandlung mit von stark ionischen Lösungen, bevorzugt HCl oder NaCl freigesetzt.

Die Isolierung der erfindungsgemäßen Peptide kann auch durch selektive Anbindung an spezielle Festphasen, bevorzugt an Festphasen, die durch Antigen-Antikörper oder ähnlich hochspezifische Wechselwirkungen eine besonders selektive Isolierung ermöglichen.

Eine weitere Methode zur Gewinnung der erfindungsgemäßen Peptide erfolgt durch die Auftrennung ihrer nativen Gemische mit Hilfe der Hochdruckflüssigkeitschromatographie (HPLC) im präparativen Maßstab. Als Fließmittel werden Acetonitril / Wasser-Gemische mit linearem Gradienten verwendet. Durch Zugabe einer starken Säure, bevorzugt Trifluoressigsäure, wird ein pH-Wert zwischen 1 und 1,5 eingestellt.

Daneben bezieht sich die vorliegende Erfindung auch auf Verfahren zur synthetischen Gewinnung der erfindungsgemäßen cysteinhaltigen Peptide und funktioneller Derivate dieser Peptide durch Peptid-Synthese. Die synthetische Herstellung der erfindungsgemäßen Peptide erfolgt durch stufenweise Verknüpfung der einzelnen Aminosäure-Bausteine. Dazu werden automatische Peptidsyntheseverfahren angewendet, wobei Festphasenverfahren und Flüssigphasenverfahren bevorzugt werden. Die Methoden sind in M. Bodanszky, Principles of Peptide Synthesis (B.M.Trost Edit.) Springer Verlag 1994, ausführlich beschrieben.

Die vorliegende Erfindung umfasst außerdem die Gewinnung der erfindungsgemäßen cysteinhaltigen Peptide durch gentechnologische Methoden. Bevorzugt wird die gentechnologische Gewinnung durch den Einbau der Gene der erfindungsgemäßen Peptide in das Erbmaterial einer Pflanze, bevorzugt Getreidesorten mit hohem Proteinertrag. Eine besonders bevorzugte Ausführungsform ist in diesem Zusammenhang der Ersatz der Thionin-Gene eines Thionin-produzierenden Getreides durch die Thionin-Gene der Pflanze Helleborus species.

Die Herstellung der erfindungsgemäßen Peptide ist auch durch transgene Exprimierung der cDNA Sequenzen möglich. Die identifizierten DNA Segmente (als cDNA Klone, als Genom-DNA Klone oder durch Oligonukleotidsynthese hergestellte DNA Segmente) können in einem biologischen System exprimiert werden. Bevorzugte biologische Systeme zur Exprimierung der erfindungsgemäßen Cystein-haltigen Peptide sind Kulturen bestimmter leicht zugänglicher Mikroorganismen wie Escherichia-coli, Pseudomonas oder Hefe.

Zur Herstellung der erfindungsgemäßen Peptide liefern auch Zellkulturen von Pflanzen, in welche die die erfindungsgemäßen Peptide dekodierenden Gensequenzen eingebaut sind, gute Ergebnisse. Für die Transformation der verwendeten Pflanzenzellen werden bekannte Methoden verwendet. Bevorzugt werden Ti-Plasmide von Agrobacterium, Elektro-Potation oder Mikroinjektion verwendet. Die genetisch veränderten Pflanzenzellen können auch als Pflanzen regeneriert werden, in welche die die erfindungsgemäßen Peptide kodierenden Gensequenzen im Genom stabil eingebaut bleiben.

Für den Einsatz der erfindungsgemäßen Peptide auf dem pharmazeutischen Gebiet können spezielle chemische Modifikationen der Peptide vorgenommen werden, durch die eine Erhöhung der Bioverfügbarkeit erreicht wird. Die Erfindung betrifft daher auch die chemische Modifikation der erfindungsgemäßen Peptide durch ihre Transformation in eine makrocyclische Verbindung, bevorzugt durch die Schließung einer zusätzlichen Bindung zwischen den zwei Aminosäuren am Anfang und am Ende der Peptid-Kette oder in der Nähe der beiden Enden. Das so hergestellte makrocyclische Peptid weist vorteilhafte physiologische Eigenschaften, insbesondere eine erhöhte Stabilität gegenüber proteolytischen und anderen Enzymen auf. Zur Herstellung der cyclischen Verbindungen werden übliche Reagenzien wie Carbodiimide, insbesondere 1-Ethyl-3-(3-dimethylaminopropyl)-carbodiimid (EDAC) verwendet.

Im Rahmen der vorliegenden Erfindung können die Hellethionine auch durch gezielten Ersatz einer oder mehreren Aminosäure-Einheiten durch eine natürliche oder synthetische Aminosäure, bevorzugt durch eine aromatische Aminosäure wie Tyrosin modifiziert werden. Eine solche Änderung der Aminosäuresequenz kann durch gezielte Mutationen des eine DNA-Sequenz der erfindungsgemäßen Peptide exprimierenden Gen-Segments erfolgen. Bevorzugt wird dabei die SPI (selective pressure incorporation) Methode (C. Minks et al. in Tetrahedron, 56 (2000) 9431-9442) angewandt.

Ein Derivat, das durch geringfügige chemische Umwandlung einer oder mehrerer Aminosäure-Bausteine der Hellethionine hergestellt werden kann, ist ebenfalls Bestandteil der vorliegenden Erfindung. Eine solche Derivatisierung führt zu einer gezielten Änderung der therapeutischen Eigenschaften der Peptide. Bevorzugt wird die Umwandlung der Hydroxy-Gruppen enthaltenden Aminosäuren wie Threonin oder Tyrosin in Ester-Derivate oder Halogen-Derivate. Die Umwandlung der freie COOH-Gruppen enthaltenden Aminosäuren in Ester-Derivate oder Amid-Derivate ist ebenfalls Bestandteil der vorliegenden Erfindung. Die Alkylierung, bevorzugt Methylierung, der freien AminoGruppen oder anderer funktioneller Gruppen führt zu einer verbesserten Bioferfügbarkeit und ist ebenfalls Bestandteil der vorliegenden Erfindung.

### Verwendung der Peptide

Die erfindungsgemäßen cysteinhaltigen Peptide, Gemische dieser cysteinhaltigen Peptide und funktionelle Derivate dieser Peptide können bei der Behandlung von Krankheiten verwendet werden, insbesondere bei der Behandlung von durch Pathogenen verursachten Krankheiten.

Daneben können die erfindungsgemäßen cysteinhaltigen Peptide, Gemische dieser cysteinhaltigen Peptide und funktionelle Derivate dieser Peptide bei der Behandlung von durch Bakterien, Fungi oder Viren verursachten Krankheiten verwendet werden.

Die erfindungsgemäßen cysteinhaltigen Peptide, Gemische dieser cysteinhaltigen Peptide und funktionelle Derivate dieser Peptide können insbesondere bei der Behandlung von Krankheiten bei Mensch und Tier, insbesondere bei größeren Tieren, bevorzugt Pferden verwendet werden.

Besondere Vorteile bringt es mit sich, die erfindungsgemäßen cysteinhaltigen Peptide, Gemische dieser cysteinhaltigen Peptide und funktionelle Derivate dieser Peptide bei der Behandlung von Krankheiten, die durch fehlerhafte Bioregulation des Immunsystems verursacht sind oder von einer fehlerhaften Bioregulation des Immunsystems begleitet werden, zu verwenden.

Außerdem können die erfindungsgemäßen cysteinhaltigen Peptide, Gemische dieser cysteinhaltigen Peptide und funktionelle Derivate dieser Peptide bei der Behandlung von Autoimmunkrankheiten, bei der Behandlung von Krebserkrankungen und bei der Behandlung von AIDS besonders erfolgreich verwendet werden.

Die vorliegende Erfindung umfasst außerdem pharmazeutische Zubereitungen, die ein oder mehrere erfindungsgemäße cysteinhaltige Peptide und/oder funktionelle Derivate dieser Peptide beinhalten. Besonders bevorzugt werden pharmazeutische Zubereitungen, die zusätzlich wenigstens ein Kohlensuboxid-Derivat umfassen.

Im Rahmen der vorliegenden Erfindung werden unter dem Begriff "Kohlensuboxid-Derivate" vom anorganischen Kohlensuboxid C₃O₂ abgeleitete Naturstoffe verstanden wie sie in der DE 196 00 301, EP 0 874 851 B1 bzw. Kerek et al., Biochim. Biophys. Acta 1567, 213-220 (2002) beschrieben sind. Die Offenbarung der DE 196 00 301 bzw. EP 0 874 851 B1 wird insoweit in die vorliegende Erfindung einbezogen. Ist also im Rahmen der vorliegenden Anmeldung von einem "Kohlensuboxid-Derivat" die Rede, so werden von diesem Begriff sämtliche in der DE 196 00 301 beschriebenen chemischen Verbindungen umfasst. Die Herstellung und Charakterisierung der Kohlensuboxid-Derivate ist ebenfalls in der DE 196 00 301 bzw. EP 0 874 851 B1 beschrieben.

Somit bezieht sich die vorliegende Erfindung insbesondere auch auf Kombinationspräparate aus mindestens einer der erfindungsgemäßen Verbindungen und mindestens einem Kohlensuboxidderivat wie vorstehend als auch in DE 196 00 301 bzw. EP 0 874 851 B1 und Kerek et al., Biochim. Biophys. Acta 1567, 213-220 (2002) beschrieben.
Von der vorliegenden Erfindung werden auch die erfindungsgemäßen cysteinhaltigen Peptide, die Gemische dieser cysteinhaltigen Peptide, die funktionellen Derivate dieser Peptide und/oder die pharmazeutisch akzeptablen Salze dieser cysteinhaltigen Peptide zur Herstellung einer pharmazeutischen Formulierung für die Behandlung von Krankheiten, insbesondere für die Behandlung von durch Pathogenen verursachten Krankheiten umfasst.

Besondere Vorteile bringt in diesem Zusammenhang die Herstellung einer pharmazeutischen Formulierung für die Prophylaxe und/oder Behandlung von Krebs mit sich, beispielsweise von Aderhautmelanom, Akute Leukämie, Akustikusneurinom, Ampullenkarzinom, Analkarzinom, Astrozytom, Basaliom, Bauchspeicheldrüsenkrebs, Blasenkrebs, Bronchialkarzinom, Brustkrebs, Burkitt-Lymphom, Corpuskarzinom, CUP-Syndrom, Dickdarmkrebs, Dünndarmkrebs, Dünndarmtumore, Eierstockkrebs, Endometriumkarzinom, Ependymom, Epithel-Krebsarten, Ewing-Tumoren, Gastrointestinale Tumoren, Gallenblasenkrebs, Gebärmutterkrebs, Gebärmutterhalskrebs, Glioblastom, Gynäkologische Tumoren, Hals-, Nasen- und Ohrentumoren, Hämatologische Neoplasien, Haarzell-Leukämie, Harnröhrenkrebs, Hautkrebs, Hirntumoren (Gliome), Hirnmetastasen, Hodenkrebs, Lymphdrüsenkrebs (Hodgkin/Non-Hodgkin), Hypophysentumor, Karzinoide, Kaposi-Sarkom, Kehlkopfkrebs, Keimzellentumor, Knochenkrebs, kolorektales Karzinom, Kopf-Hals-Tumore, Kolonkarzinom, Kraniopharyngeome, Krebs im Mundbereich und auf der Lippe, Leberkrebs, Lebermetastasen, Leukämie, Lidtumor, Lungenkrebs, Lymphome, Magenkrebs, Malignes Melanom, Mammakarzinom, Mastdarmkrebs, Medulloblastome, Melanom, Meningeome, Morbus Hodgkin, Mycosis fungoides, Nasenkrebs, Neurinom, Nierenkrebs, Non-Hodgkin-Lymphome, Oligodendrogliom, Ösophaguskarzinom, Osteosarkom, Ovarial-Karzinom, Pankreaskarzinom, Peniskrebs, Plasmozytom, Prostatakrebs, Rachenkrebs, Rektumkarzinom, Retinoblastom, Scheidenkrebs, Schilddrüsenkarzinom, Schneeberger Krankheit, Speiseröhrenkrebs, Spinaliom, T-Zell-Lymphom (Mycosis fungoides), Thymom, Tubenkarzinom, Tumoren des Auges, Urethrakrebs, Urologische Tumoren, Urothelkarzinom, Vulvakrebs, Warzenbeteiligung, Weichteiltumoren, Wilms Tumor, Zervixkarzinom und Zungenkrebs.

Vorzugsweise handelt es sich bei der Krebsart um eine ausgewählt aus der Gruppe bestehend aus Blasenkrebs, Brustkrebs, Krebs des zentralen Nervensystems, Dickdarmkrebs, Magenkrebs, Lungenkrebs, Hautkrebs, Kopf- und Nackenkrebs, Eierstockkrebs, Gebärmutterhalskrebs, Glioblastomkrebs, Prostatakrebs, Hodenkrebs, Leukämie, Leberkrebs, Nierenkrebs und Epithel-Krebsarten handelt.

Ferner ist Gegenstand der Erfindung auch Kombinationspräparate aus mindestens einer vorgenannten erfindungsgemäßen Verbindung zusammen mit einem Zytostatika. Als Zytostatika kommen Alkylierungsmittel, Antibiotika mit zytostatischen Eigenschaften, antimetabolische Wirkstoffe, Alkaloide, Podophyllotoxine, Platin-enthaltende Verbindungen, Taxane, zytostatische Wirkstoffe und monoklonale Antikörper in Frage. Beispiele für diese Verbindungsklassen sind beispielsweise Cyclophosphamid, Ifosfamid, Trofosfamide, Temozolomid, Chlorambucil, Melphalan, Busulfan, Treosulfan, Thiotepa, Estramustin, Nimustin, Carmustin, Lomustin, Dacarbazin, Procarbazin, Adriamycin (Doxorubicin), Epirubicin (4-Epi-Adriamycin), Idarubicin, Actinomycin D, Daunorubicin, Bleomycin, Dactinomycin, Mitomycin C, Mitoxantron, Methotrexat, 5-Fluorouracil, Capecitabin, Cytosinarabinosid, Tioguanin, Mercaptopurin, Fludarabin, Cladribin, Gemcitabin, Vincristin, Vinblastin, Vindesin, Etoposid, Teniposid, Cisplatin, Carboplatin, Oxaliplatin, Paclitaxel, Docetaxel, Hydroxycarbamide (Hydroxyurea), Imatinib, Miltefosin, Amsacrin, Topotecan (Topoisomerase-1-Inhibitor), Pentostatin, Bexaroten, Tretinoin, Asparaginase, Trastuzumab (Herceptin®), Alemtuzumab (MabCampath®), Rituximab (MabThera®).

Die vorliegende Erfindung umfasst auch Arzneimittel, die ein oder mehrere erfindungsgemäße cysteinhaltige Peptide und/oder funktionelle Derivate dieser Peptide enthalten. Besonders bevorzugt werden Arzneimittel, die zusätzlich wenigstens ein Kohlensuboxid-Derivat und/oder eine zytostatische bzw. zytotoxische Verbindung enthalten.

Die erfindungsgemäßen cysteinhaltigen Peptide können sowohl als Abwehrstoff gegen Pathogene als auch als pharmazeutischer Wirkstoff zur Bekämpfung der von den Pathogenen verursachten Infektionen und Krankheiten verwendet werden. Insbesondere ist der Einsatz gegen Krankheiten möglich, die durch eine chronisch fehlerhafte lmmunregulation gekennzeichnet sind wie z.B. Autoimmunkrankheiten, Krebs oder AIDS. Die erfindungsgemäßen cysteinhaltigen Peptide können separat als einzelne Substanz, aber auch als Gemisch mehrerer Peptide oder zusammen mit anderen bereits bekannten Wirkstoffen und Trägermaterialien eingesetzt werden.

Die erfindungsgemäßen Kombinationspräparate als auch die erfindungsgemäßen pharmazeutischen Formulierungen, welche mindestens ein erfindungsgemäßes Peptid enthalten, werden mit den üblichen festen oder flüssigen Trägerstoffen oder Verdünnungsmitteln und den üblicherweise verwendeten pharmazeutischen Hilfsstoffen entsprechend der gewünschten Applikationsart mit einer geeigneten Dosierung in bekannter Weise hergestellt. Die bevorzugten pharmazeutischen Formulierungen oder Zubereitungen bestehen in einer Darreichungsform, die zur oralen Applikation bzw. zur Inhalation geeignet ist. Solche Darreichungsformen sind beispielsweise Tabletten, Filmtabletten, Schichttabletten, Dragees, Kapseln, Mikrokapseln, Pillen, Granulaten, Pulver, Puder, Lösungen, Dispersionen, Suspensionen Suppositorien, Emulsionen, Dispersionen, Zäpfchen, Gelen, Salben, Sirup oder Depotformen oder Inhalationslösungen bzw. Inhalationspulver. Zudem umfassen die erfindungsgemäßen pharmazeutischen Zusammensetzungen Formulierungen wie Schichttabletten zur kontrollierten und/oder kontinuierlichen Freisetzung des Wirkstoffs sowie Mikroverkapselungen als spezielle Darreichungsform.

Derartige pharmazeutische Zubereitungen sind unter anderem für die Inhalation oder die intravenöse, intraperitoneale, intramuskuläre, subkutane, mucokutane, orale, rektale, transdermale, topikale, intradermale, intragastrale, intrakutane, intravaginale, intranasale, intrabuccale, perkutane oder sublinguale Verabreichung geeignet. Besonders vorteilhafte Darreichungsformen sind die orale Applikation, Injektion als auch Inhalation.

Entsprechende Tabletten können beispielsweise durch Mischen der erfindungsgemäß einsetzbaren Verbindung und/oder deren Salz mit bekannten Hilfsstoffen, beispielsweise inerten Verdünnungsmitteln wie Dextrose, Zucker, Sorbit, Mannit, Polyvinylpyrrolidon, Sprengmitteln wie Maisstärke oder Alginsäure, Bindemitteln wie Stärke oder Gelantine, Gleitmitteln wie Magnesiumstearat oder Talk und/oder Mitteln zur Erzielung eines Depoteffektes wie Carboxypolymethylen, Carboxymethylcellulose, Celluloseacetatphthalat oder Polyvinylacetat, erhalten werden. Die Tabletten können auch aus mehreren Schichten bestehen.

Entsprechend können Dragees durch Überziehen von analog den Tabletten hergestellten Kernen mit üblicherweise in Drageeüberzügen verwendeten Mitteln, beispielsweise Polyvinylpyrrolidon oder Schellack, Gummiarabicum, Talk, Titandioxid oder Zucker hergestellt werden. Dabei kann auch die Drageehülle aus mehreren Schichten bestehen, wobei die oben bei den Tabletten erwähnten Hilfsstoffe verwendet werden können.

Lösungen oder Suspensionen mit dem erfindungsgemäß einsetzbaren Wirkstoff können zusätzlich geschmacksverbessernde Mittel wie Saccharin, Cyclamat oder Zucker sowie Aromastoffe wie Vanillin oder Orangenextrakt enthalten. Sie können außerdem Suspendierhilfsstoffe wie Natriumcarboxymethylcellulose oder Konservierungsmittel wie p-Hydroxybenzoate enthalten. Wirkstoffe enthaltende Kapseln können beispielsweise hergestellt werden, indem man den Wirkstoff mit einem inerten Träger wie Milchzucker oder Sorbit mischt und in Gelatinekapseln einkapselt.

Geeignete Suppositorien lassen sich beispielsweise durch Vermischen mit dafür vorgesehenen Trägermitteln wie Neutralfetten oder Polyethylenglykol bzw. Derivaten davon herstellen.

Derartige Formulierungen sind unter anderem für die Inhalation oder die intravenöse, intraperitoneale, intramuskuläre, subkutane, mucokutane, orale, rektale, transdermale, topikale, intradermale, intragastrale, intrakutane, intravaginale, intranasale, intrabuccale, perkutane oder sublinguale Verabreichung geeignet.

Als pharmakologisch verträgliche Träger können beispielsweise Lactose, Stärke, Sorbitol, Sucrose, Cellulose, Magnesiumstearat, Dicalciumphosphate, Calciumsulfate, Talk, Mannitol, Ethylalcohol und dergleichen eingesetzt werden. Puder als auch Tabletten können zu 5 bis 95% aus einem derartigen Träger bestehen.

Als Bindemittel können zudem Stärke, Gelatine, natürliche Zucker, natürliche als auch synthetische Gummis wie beispielsweise Akaziengummi oder Guar-Gummi, Natriumalginat, Carboxymethyl-Cellulose, Polyethylenglycol und Wachse eingesetzt werden. Als Gleitmittel können Borsäure, Natriumbenzoat, Natriumacetat, Natriumchlorid, und dergleichen dienen.

Ferner können den pharmazeutischen Zusammensetzungen noch Sprengmittel, Farbstoffe, Geschmacksstoffe und/oder Bindemittel zugesetzt werden.

Flüssige Formulierungen umfassen Lösungen, Suspensionen, Sprays und Emulsionen. Beispielsweise Injektionslösungen auf Wasserbasis oder Wasser-Propylenglycol-Basis für parenterale Injektionen.

Für die Zubereitung von Suppositorien werden bevorzugt niedrigschmelzende Wachse, Fettsäureester und Glyceride eingesetzt.

Kapseln werden beispielsweise aus Methylcellulose, Polyvinylalcohole oder denaturierte Gelatine oder Stärke hergestellt.

Als Sprengmittel können Stärke, Natrium-Carboxymethylstärke, natürliche und synthetische Gummis wie beispielsweise Johannisbrotkernmehl, Karaya, Guar, Tragacanth und Agar sowie Cellulosederivate wie Methylcellulose, Natrium-Carboxymethylcellulöse, microkristalline Cellulose sowie Alginate, Tonerden und Bentonite verwendet werden. Diese Bestandteile können in Mengen von 2 bis 30 Gew.-% eingesetzt werden.

Als Bindemittel können Zucker, Stärke aus Korn, Reis oder Kartoffeln, natürliche Gummis wie Akaziengummi, Gelatine, Tragacanth, Alginsäure, Natriumalginat, Ammonium-Calcium-Alginat, Methylcellulose, Natrium-Carboxymethylcellulose, Hydroxypropylmethylcellulose, Polyvinylpyrrolidon sowie anorganische Verbindungen wie Magnesium-Aluminum-Silicate zugesetzt werden. Die Bindemittel könne in Mengen von 1 bis 30 Gew.-% zugesetzt werden.

Als Gleitmittel können Stearate wie Magnesiumstearat, Calciumstearat, Kaliumstearat, Stearinsäure, hochschmelzende Wachse als auch wasserlösliche Gleitmittel wie Natriumchlorid, Natriumbenzoat, Natriumacetat, Natrioumoleat, Polyethylenglycol und Aminosäuren wie Leucin eingesetzt werden. Derartige Gleitmittel können in Mengen von 0,05 bis 15 Gew.-% verwendet werden.

Eine bevorzugte Anwendung der erfindungsgemäßen cysteinhaltigen Peptide besteht in der Unterstützung der Abwehr biologischer Organismen, insbesondere Pflanzen, gegenüber bakteriellen, fungalen, viralen oder anderen Pathogenen. Diese Anwendung kann durch Zuführung des Peptids in die von der Pflanze aufgenommenen Nährstoffe /Flüssigkeiten oder durch Aufbringung der das Peptid enthaltenden Lösung auf die Oberfläche der Pflanzenblätter erfolgen.

Von der vorliegenden Erfindung umfasst ist auch der genetische Einbau von Gensequenzen, die ein erfindungsgemäßes Peptid exprimieren, in das Genom eines von einer Krankheit bedrohten Organismus, bevorzugt in das Genom von Pflanzen. Die erhöhte Resistenz des durch diese genetische Änderung erzeugten neuen Organismus bietet einen für Mensch und Umwelt deutlich schonenderen Schutz gegen Pathogene als die bisher bekannten chemischen Pestizide.

Die erfindungsgemäßen Peptide können in ähnlicher Weise zur präventiven Stärkung der Abwehrkräfte anderer, insbesondere tierischer Organismen gegenüber Pathogen-Infektionen unterschiedlicher Art eingesetzt werden. Durch diese Art der Anwendung werden Pathogen-tragende Insekten, Nematoden und andere erfolgreich bekämpft. Dadurch wird die Übertragung bzw. Freisetzung der Pathogene sehr effizient verhindert und dies eventuell sogar noch bevor deren krankheitserregende Wirkung sich entfalten kann.

Eine weitere Anwendung der erfindungsgemäßen Peptide erfolgt durch Applikation an einem mit Pathogen bereits infizierten biologischen, insbesondere tierischen Organismus. Dadurch werden die schädlichen Folgen der bakteriellen, viralen oder anderer Infektionen neutralisiert oder zumindest minimiert. Zu diesem Zweck wird das Peptid oder das Peptidenthaltende Gemisch so eingesetzt, dass es zusammen mit den eigenen Abwehrkräften des Organismus eine deutlich wirksamere Bekämpfung des Pathogens bewirkt.

Die erfindungsgemäßen Peptide führen bei Menschen und Tieren zu einer signifikanten Herabsetzung der Exprimierung einiger pro-inflammatorischen Zytokine, insbesondere von IL-2, IL-3, IL-4 und γ-IFN. Die erfindungsgemäßen Peptide sind also fähig die gegen das eigene Gewebe gerichteten auto-aggressiven Vorgänge, insbesondere bei Autoimmunkrankheiten, signifikant zu reduzieren.

Durch Stimulierung von inhibitorischen Zytokinen, insbesondere von TGF beta sind die erfindungsgemäßen Peptide in der Lage, die Aktivität der bei Autoimmunkrankheiten pathologisch überaktivierten primären humanen Immunzellen zu verringern. Besonders vielversprechende experimentelle Ergebnisse wurden bei topischer Anwendung der erfindungsgemäßen Peptide bei Hautkrankheiten mit Autoimmun-Charakter, insbesondere bei der Behandlung von Psoriasis, erhalten.

Durch die Stimulierung der Produktion des suppressiv und gegebenenfalls regulatorisch wirkenden Zytokins IL-10 wird durch die erfindungsgemäßen Peptide das normale Gleichgewicht zwischen den pro- und anti-inflammatorischen Zytokinen, das bei Autoimmunkrankheiten und anderen chronischen Erkrankungen außer Kontrolle gerät, wiederhergestellt.

Die erfindungsgemäßen Peptide üben eine effiziente und zum Teil selektive Hemmung auf die Vermehrung maligner Krebszellen aus. Dies konnte durch Zellkulturexperimente bestätigt werden.

Von der vorliegenden Erfindung werden auch pharmazeutische Zubereitungen und Arzneimittel umfasst, die ein erfindungsgemäßes Peptid oder ein Gemisch erfindungsgemäßer Peptide als wirksamen Anteil beinhalten. Weiterhin sind pharmazeutische Zusammensetzungen und Arzneimittel umfasst, die ein erfindungsgemäßes Peptid oder ein Gemisch erfindungsgemäßer Peptide zusammen mit wenigstens einem, bereits bekannten Wirkstoff und/oder zusammen mit pharmazeutisch verträglichen und annehmbaren Verbindungen und Trägern enthalten.

Die Verwendung eines erfindungsgemäßen Peptids zusammen mit den als MCS bezeichneten Kohlensuboxid-Derivaten führt zu einer deutlich verbesserten Bioverfügbarkeit und immunregulatorischen Wirksamkeit des Peptids, insbesondere bei onkologischen Anwendungen (siehe Beispiele).

Zur Erhöhung der Selektivität bei der Bekämpfung maligner Tumorzellen können die erfindungsgemäßen Peptide mit tumorspezifischen Antigenen gekoppelt werden. Gemäß einer bevorzugten Ausführungsform wird das Peptid mit der Sequenz HT-C1 an den gegen humane Prostata-Karzinomzellen gebildeten und durch Affinitätschromatographie isolierten Antikörper gebunden. Die Bindung erfolgt mit Hilfe von bekannten Kopplungsreagenzien wie Carbodiimiden, bevorzugt mit dem wasserlöslichen 1-Ethyl-3-(3-dimethylaminopropyl)-carbodiimid (EDAC).

Gegenüber der Verwendung einzelner erfindungsgemäßer Peptide bietet die Verwendung eines Gemisches der erfindungsgemäßen Peptide deutliche Vorteile. Im Vergleich zur Anwesenheit von nur einem Agens erschwert nämlich ein Gemisch von mehreren Wirkstoffen die Resistenz-Bildung des Pathogens. Die selben Vorteile ergeben sich sowohl bei der biologischen Abwehr durch die transgene Exprimierung des Peptidgemisches als auch bei der Krankheitsbekämpfung durch Verabreichung der Peptide an den bereits erkrankten Organismus.

Die erfindungsgemäßen Peptide können also als Wirkstoff auf die oben beschriebenen Arten eingesetzt werden. Neben der Verwendung der einzelnen erfindungsgemäßen Peptide werden die erfindungsgemäßen Peptide als Gemisch mehrerer Peptide ebenso eingesetzt wie zusammen mit anderen Substanzen, insbesondere zusammen mit Kohlensuboxid-Derivaten. Als Darreichungsform kommen Injektionen, Spray und eine topische Applikation in Frage.

Die erfindungsgemäßen Peptide werden bei jeder Art von Applikation in einer 0,0001 bis 10%igen Lösung, insbesondere in einer 0,01 bis 1 %igen Lösung, besonders bevorzugt in einer ungefähr 0,2%igen Lösung eingesetzt. Besonders bevorzugt wird die Verwendung eines wässrigen Peptidgemisches. Die Behandlung kann z.B. durch Gabe von täglich 3 x 10 ml der wässrigen Peptid-Lösung erfolgen.

Die erfindungsgemäßen Peptide können den zu behandelnden Menschen, Tieren oder Pflanzen als Reinstoff verabreicht werden oder als pharmazeutische Zusammensetzung, wobei sie im Gemisch mit Trägerstoffen oder Verdünnungsmitteln in therapeutisch effektiver Dosierung verabreicht werden. Solche therapeutisch effektiven Dosen können separat oder in Verbindung mit anderen therapeutischen Verbindungen verabreicht werden. Bei der Verwendung zur Behandlung von Krebs kommen in diesem Zusammenhang anti-proliferative und anit-angiogene Agenzien wie zytostatische oder zytotoxische Anti-Tumor-Agenzien wie z.B. 5FU, Cisplatin in Frage oder auch Proteinkinaseinhibitoren wie der Flk-1/KDR Inhibitor CGP 79787, die Verbindungsklasse der Indolocarbazole, wie z.B. Gö7612, die Verbindungsklasse der Bisindolymaleimide, wie z.B. LY 333531, GF109203x, Ro 32-0432, Ro 31-8220, die Verbindungsklasse der Balanol-Derivate, wie z.B. SPC 100840, die Verbindungsklasse der Antisense-Oligodeoxinukleotide, wie z.B. CGP 64128A und VEGF Antisense Oligonukleotid, die Verbindungsklasse der Alkyl-lysophospholipide, wie z.B. ET-18-OCH3, Inhibitoren der Wachstumsfaktor-Rezeptoraktivierung wie anti-HER2/neu Antikörper, Trastuzumab (Herceptin), Inhibitoren der Wachstumsfaktor-Rezeptor-Kinaseaktivität wie die Verbindungsklasse der Phenylaminochinazoline, wie z.B. PQ 153035, ZD 1839 und CP-358774, die Verbindungsklasse der substituierten Pyrimidine umfassend Pyrido-, Pyrrolo-, Pyrazolo-, Pyrimido- und Phenylaminopyrimidine, wie z.B. PD 158780, PD 166285, CGP 59326, CGP 60261 und CGP 62706, die Verbindungsklasse der Tyrphostine, wie z.B. AG1478, RG 13022 und AG 825, die Verbindungsklasse der Lavendustine, wie z.B. Lavendustin A, die Verbindungsklasse der Dianilinophthalimide, wie z.B. CGP54698, sowie Verbindungen ausgewählt aus der Gruppe bestehend aus Inhibitoren von MAPKKK, Inhibitoren von MAPKK, Inhibitoren von MAPK wie z.B. PD098059, U0126 oder SB203580, Interferon alpha, rekombinanter Faktor 4 für Blutplättchen, Angiostatin oder die polyanionische Verbindung Suramin. Die vorliegende Erfindung umfasst auch die Kombination der erfindungsgemäßen Peptide mit einer der oben angegebenen Verbindungen.

Techniken für die Formulierung und Gabe der erfindungsgemäßen Peptide können in "Remington's Pharmaceutical Sciences" Mack Publishing Co., Easton PA gefunden werden. Eine Zusammensetzung, die eines der erfindungsgemäßen Peptide umfasst, kann in Form einer Lösung des erfindungsgemäßen Peptids in einem flüssigen pharmazeutischen Träger oder in jeder anderen Formulierung wie Tabletten, Pillen, Dragees, Kapseln, Gel, Sirup, Brei, Suspension und ähnlichem vorliegen.

### Kurze Beschreibung der Zeichnungen

Die Erfindung soll nachfolgend anhand von Ausführungsbeispielen im Zusammenhang mit den Figuren näher erläutert werden. Es zeigen
- Fig. 1: die Struktur des Hellethionins HT-D;
- Fig. 2: ein HPLC-Diagramm eines Hellethionin-Gemisches;
- Fig. 3: die Wirkung unterschiedlicher Konzentrationen des Hellethionins HT-C auf Brustkrebszellen des Typs MCF-7.

### Wege zur Ausführung der Erfindung

### Beispiel 1: Gewinnung eines Peptid-Gemisches

Rund 10 kg grob gemahlene Wurzel und Wurzelstock von *Helleborus niger* (Fam. Ranunculaceae) werden 6 Stunden mit 50 I TBM-Hexan-Gemisch (1:1) behandelt. Das entfettete und an Luft getrocknete Pflanzenmaterial wird zweimal mit jeweils 60 I EtOH 50% ca. 24 Stunden unter leichtem Rühren bei Zimmertemperatur extrahiert. Die vereinten alkoholisch-wässrigen Lösungen werden im Vakuum-Rotationsverdampfer bei 70 °C bis zur Trockene eingeengt. Der Trockenrückstand wird dreimal mit je 3 10,05 N Salzsäure behandelt, die resultierenden wässrigen Emulsionen werden vereint und sukzessiv mit je 10 I Hexan, Chloroform und TBM extrahiert. Die wässrige Phase wird im Vakuum auf ca. 10 I Volumen eingeengt und die Lösung mit ca. 200 g Aktivkohle behandelt (2 Stunden). Das Filtrat wird im Vakuum bis auf 1,0 I eingeengt. Das wässrige Konzentrat wird mit Hilfe einer 1 N HCl Lösung auf den pH-Wert 1,2 eingestellt und unter starkem Rühren in das zehnfache Volumen gekühltes (10 °C) Aceton eingegossen. Der sich bildende weiße Niederschlag wird durch Zentrifugation von dem Überstand getrennt und im Vakuum getrocknet. Der trockene Niederschlag wird anschließend in einer minimalen Menge Wasser aufgelöst und in ein ca. zehnfaches Volumen gekühltes Aceton unter Rühren eingegossen. Dieser Präzipitations-Vorgang wird noch einmal wiederholt, das erhaltene Gemisch der cysteinhaltigen Peptide in Wasser aufgelöst und liophilisiert.

Das Gemisch der erfindungsgemäßen Peptide wird durch HPLC charakterisiert (Figur 2). Dazu wurde eine Nucleosil 100-7, C-18 Säule (Macherey-Nagel Düren) mit 250 mm Länge und 21 mm ID verwendet. Bei einer Flussrate von 3 ml/min und einem linearen Elutionsgradienten von 20 - 50% Acetonitril in 25 min. eluieren die erfindungsgemäßen Peptide wie folgt:

| | |
|---|---|
| Hellethionin-A | 14,4 min. |
| Hellethionin-B1 bis Hellethionin-B6 | 16,1 min. |
| Hellethionin-C | 16,9 min. |
| Hellethionin-D | 18,3 min. |
| Hellethionin-E1, Hellethionin-E2 | 20,1 min. |

### Beispiel 2: Gewinnung des reinen Peptids HT-D

Rund 1 kg gemahlene Wurzeln der Pflanze *Helleborus purpurascens* werden 4 Stunden mit 10 I verdünnter Essigsäure (5% in Wasser) bei 40°C extrahiert. Die filtrierte Lösung wird im Vakuum bis auf 1 I eingeengt. Danach werden in der Lösung ca. 400 g Ammoniumsulfat gelöst. Der sich bildende Niederschlag wird durch Zentrifugieren abgetrennt, in einer minimalen Menge Wasser aufgelöst und die Lösung zu einem Gemisch von 3,6 I Aceton und 1,4 I Ethanol unter kräftigem Rühren zugefügt. Die Bildung des Niederschlags im Aceton / Ethanol-Gemisch wird noch zweimal wiederholt und das erhaltene Peptid-Rohgemisch in Wasser aufgelöst und liophilisiert.

Danach werden 5 g liophilisiertes Rohgemisch des Peptids aus der Gattung *Helleborus* in 100 ml 20% Acetonitril / Wasser mit 0,1% Gehalt an Trifluoressigsäure aufgelöst und mit Hilfe der präparativen Hochdruckflüssigkeitschromatographie (HPLC) in einzelne Komponenten aufgetrennt. Zu diesem Zweck wird die Probelösung in aliquoten Mengen auf eine Nucleosil 100-7, C-18 Säule (Macherey-Nagel Düren) mit 250 mm Länge und 21 mm ID (Innen-Durchmesser) aufgetragen. Die Trennung erfolgt bei einer Flussrate von 3 ml/min mit Hilfe eines linearen Elutionsgradienten von 20 - 50% Acetonitril in 30 Minuten. Die einzelnen reinen Peptide werden mit Hilfe eines Probensammlers vom Typ FRAC-100 der Fa. Pharmacia-Biotech aufgefangen. Das reine Peptid HT-D wird in dem Retentionszeitbereich von 17,9 - 18,7 min. aufgefangen.

Die Prüfung auf Reinheit des isolierten Peptids Hellethionin-D erfolgt durch analytische HPLC mit Hilfe einer "Luna-CN" Säule der Fa. Phenomenex (Offenbach) mit 200 mm Länge und 4 mm Durchmesser unter Verwendung eines linearen Gradienten von 5% bis 85 % Acetonitril in 40 Minuten.

Das ¹H NMR Spektrum von reinem Hellethionin-D in Wasser wurde mit der durch Edman-Abbau bestimmten Aminosäure-Sequenz korreliert und die einzelnen Signale mit Hilfe von NOESY- und TOCSY-Spektren zugeordnet. In der Tabelle 1 sind die durch diese Korrelation erhaltenen einzelnen Resonanz-Signale für HT-D dargestellt.

**Tabelle 1: ¹H NMR Signalzuordnungen für Hellethionin-D**

| Position | Aminosäure | H - N | H - C alpha | H - Cbeta | H - C gamma | H - C delta | andere |
|---|---|---|---|---|---|---|---|
| 1 | Lys | | 3,72 | 1,36-1,47 | 0,83 | 1,1-1,03 | HE: 2,64 HZ: 7,17 |
| 2 | Ser | 8,52 | 4,65 | 3,41-2,96 | | | |
| 3 | Cys | 8,45 | 4,6 | 4,22-1,93 | | | |
| 4 | Cys | 9,47 | 4,9 | 2,60-2,14 | | | |
| 5 | Arg | 7,52 | 3,51 | 1,65 | 1,35-1,30 | 2,93-2,62 | H-E: 7,00 |
| 6 | Asn | 6,82 | 4,46 | 3,01 | 7,51-6,45 | | |
| 7 | Thr | 8,3 | 3,58 | 3,79 | 0,94 | | |
| 8 | Leu | 7,65 | 3,77 | 1,38-1,29 | 0,57-0,61 | | |
| 9 | Ala | 8,02 | 4,07 | 1,52 | | | |
| 10 | Arg | 7,68 | 4,16 | 1,92-1,36 | 1,59 | 3,06-3,30 | HE: 8,07; HH: 6,10 |
| 11 | Asn | 8,16 | 4,21 | 2,64-2,60 | 6,7-7,29 | | |
| 12 | Cys | 8,15 | 3,89 | 3,58-2,72 | | | |
| 13 | Tyr | 8,74 | 3,36 | 3,06-2,89 | | | HE: 6,40 |
| 14 | Asn | 8,55 | 4 | 2,62-2,52 | 7,6 | | |
| 15 | Ala | 7,63 | 3,93 | 1,19 | | | |
| 16 | Cys | 7,98 | 3,8 | 2,83-2,78 | | | |
| 17 | Arg | 8,26 | 3,79 | 1,48-1,37 | 0,88-0,62 | 2,72-2,15 | HE: 6,72; HH: 6,8-6,3 |
| 18 | Phe | 8 | 4,01 | 3,07-2,94 | | HD; HE; HZ: 7,01 - 6,91 | |
| 19 | Thr | 7,24 | 3,94 | 4,27 | 1,06 | | |
| 20 | Gly | 7,24 | 4,04-3,30 | | | | |
| 21 | Gly | 7,88 | 3,73-3,12 | | | | |
| 22 | Ser | 8,45 | 4,03 | 3,90-3,74 | | | |
| 23 | Gln | 9,01 | 3,76 | 1,84-1,73 | 2,20-1,95 | 1,95 | HE: 7,1-6,58 |
| 24 | Pr | | 4,05 | 1,99-1,56 | 1,78-1,65 | 3,54-3,39 | |
| 25 | Thr | 7,1 | 3,55 | 3,8 | 0,84 | | |
| 26 | Cys | 8,4 | 4,28 | 2,29-2,16 | | | |
| 27 | Gly | 8,42 | 3,83-3,45 | | | | |
| 28 | Ile | 7,44 | 3,66 | 1,67 | 1,41-1,01 | 0,56 | |
| 29 | Leu | 7,81 | 3,81 | 1,54-1,45 | 1,28 | 0,57 | |
| 30 | Cys | 7,4 | 4,51 | 3,54-2,65 | | | |
| 31 | Asp | 7,7 | 4,27 | 3,36-2,62 | | | |
| 32 | Cys | 8,84 | 5,24 | 2,64-1,79 | | | |
| 33 | Ile | 8,64 | 4,25 | 1,4 | 0,57-0,7 | -0,01 | |
| 34 | His | 8,52 | 4,82 | 2,73-2,69 | 8,53 | | |
| 35 | Val | 7,96 | 4,44 | 1,99 | 0,44-0,39 | | |
| 36 | Thr | 8,54 | 4,15 | 4,21 | 0,89 | | |
| 37 | Thr | 6,65 | 4,17 | 4,31 | 0,91 | | |
| 38 | Thr | 8,22 | 3,82 | 4,02 | 0,93 | | |
| 39 | Thr | 6,78 | 4,16 | 3,79 | 0,82 | | |
| 40 | Cys | 8,57 | 4,59 | 3,55-2,14 | | | |
| 41 | Pro | | 4,29 | 1,98-1,84 | 1,5 | 3,51-3,30 | |
| 42 | Ser | 8,55 | 3,94 | 3,71-3,59 | | | |
| 43 | Ser | 7,62 | 3,87 | 3,76-3,65 | | | |
| 44 | His | 7,41 | 4,37 | 2,21-2,01 | 6,72 | | HE: 8,44 |
| 45 | Pro | | 4 | 1,66 | 1,66 | 3,42-3,07 | |
| 46 | Ser | 7,78 | 4,04 | 3,42-2,56 | | | |

### Beispiel 3: Wirkung des Peptids HT-A auf die Zytokin-Produktion von primären humanen Immunzellen

Die Wirkung des Peptids HT-A auf die Zytokin-Produktion von primären humanen Immunzellen wurde durch die Messung der Konzentration von Zytokinen in aus humanen Blut gewonnenen Lymphozyten-Kulturen ermittelt. Es wurden Dosierungen von 4 - 200 µg/ml der Hellethionine angewendet, die durch Zugabe der errechneten Mengen in Form einer Stammlösung erhalten wurden. Die Konzentration einzelner Zytokine in den Mess- bzw. Kontrollproben wird mit Hilfe der kommerziell erhältlichen "Quantikine" ELISA Platten der Fa. R&D Biosystems, Minneapolis, USA, ermittelt. Die Ergebnisse wurden mit Kontrollproben, d.h. mit der Lymphozytenkultur ohne Zugabe des Peptids verglichen.

Bei den mit 4 µg bzw. 200 µg Peptid HT-A behandelten Lymphozytenkulturen (4 Millionen Zellen pro ml) wurden die folgende Zytokin (bzw. Zytokin-Rezeptor) Konzentrationen [pg/ml] im Vergleich mit der Kontrollprobe (ohne Peptid) erhalten:

| **Zytokin/Peptid** | **Kontrolle** | **HT-A** | **HT-A** |
|---|---|---|---|
| | [ohne Peptid] | [4 µg/ml] | [200 µg/ml] |
| | | | |
| *gehemmt* | | | |
| **IL-2** [pg/ml] | 9.390 | 1.940 | 1.855 |
| **IL-3** [pg/ml] | 41 | 26 | 8 |
| **IL-4** [pg/ml] | 34 | 19 | 12 |
| **γ-IFN** [pg/ml] | 11.605 | 7.454 | 7.504 |
| **IL-6R** [pg/ml] | 171 | 100 | 65 |
| | | | |
| *stimuliert* | | | |
| **IL-10** | 69 | 288 | 12 |
| **IL-2R** | 44 | 30 | 67 |
| **TGF-β2** | 9 | 105 | 45 |
| | | | |
| *nicht beeinflusst* | | | |
| **IL-6** | 1.095 | 1.100 | 1.130 |
| **IL-1RA** | 2.170 | 2.204 | 2.467 |
| **TNF-α** | 2.105 | 1.860 | 2.085 |

Diese Daten zeigen, dass das Peptid HT-A die Exprimierung von mehreren, proinflammatorisch wirkenden Zytokinen wie z.B. IL-2, IL-3, IL-4 und γ-IFN hemmt. Durch Unterdrückung der Produktion dieser Zytokine führt die Verwendung des Peptids zur erwünschten Reduzierung der schädlichen autoaggressiven Vorgänge autoimmuner Krankheiten.

Darüber hinaus zeigt das Peptid HT-A eine Stimulierung der suppressiv wirkenden Zytokine wie z.B. IL-10 und TGF-β2. Interessanterweise ist die Stimulierung dieser Zytokine bei geringer Peptid-Konzentration (4 µg/ml) deutlich intensiver als bei höherer Dosierung (200 µg/ml). Es wird vermutet, dass bei höher Peptid-Konzentration die unspezifische zelluläre Toxizität des Peptids die bei kleineren Dosen beobachtete Stimulation überdeckt.

Das untersuchte Peptid HT-A zeigt auf die Produktion einiger Zytokine wie z.B. IL-6 oder TNF-α und auf die Exprimierung des IL-1 Rezeptors keinen signifikanten Einfluss.

### Beispiel 4: Wirkung des Peptids HT-C auf die Proliferation von humanen Krebszellen

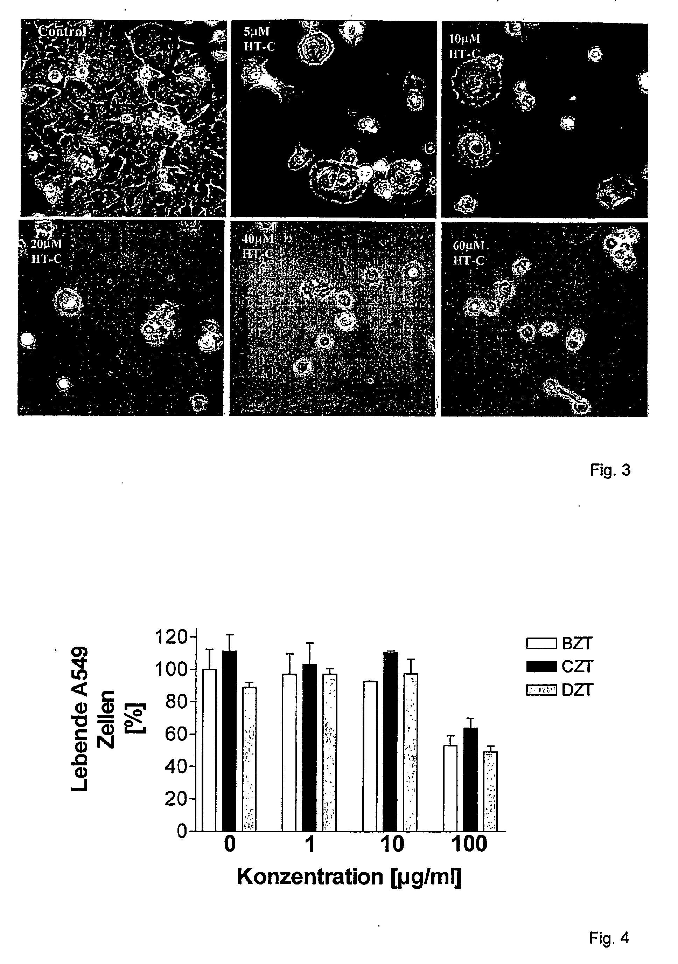

Die Untersuchungen wurden an der Brustkrebs-Zellkulturlinie MCF-7 durchgeführt. Als Vergleich diente eine Zellkulturlinie aus nicht differenzierten Brust-Epithel-Zellen des Typs MCF-10A. Die entsprechende Zellen (ca. 10⁵ /ml Probe) wurden in dem Standard DMEM Kulturmedium zunächst stimuliert. Nach 24 Stunden wurde das Peptid HT-C zugefügt und auf diese Weise Konzentrationen zwischen 0,2 und 400 µg/ml eingestellt. Die Prüfung der von dem Peptid verursachten Veränderungen erfolgte in 24 Stunden-Intervallen in einem Zeitraum von bis zu 6 Tagen. Das Peptid HT-C verursacht bereits bei geringen Konzentrationen, nämlich schon ab 2 µg/ml eine sehr deutliche Hemmung der Vermehrung der MCF-7 Zellen (Figur 3).

Das Peptid HT-C hatte unter den selben Bedingungen eine signifikant geringere Hemmung auf die Vermehrung der nicht malignen Epithel-Zellen von Typ MCF-10.

### Beispiel 5: Wirkung der Peptid-Gemische auf die Tumorentwicklung bei Mäusen

Bei der Studie wurde die Wirkung der erfindungsgemäßen Peptide im Gemisch auf die Tumorentwicklung der mit WAZ-2T Zellen inokulierten weiblichen Mäuse des Typs BALB/c mit einem Körpergewicht von 20 bis 24 Gramm untersucht. Zunächst wurde die Pathogenität des Zellstammes durch die Inokulation einer steigenden Dosis von 0 bis 10⁶ Tumorzellen in den rechten Brust-Fettpolster der Tiere (12 Mäuse / Dosis) ermittelt. Dieser Vorversuch ergab, dass die Verwendung einer Dosis von 2,5 x 10³ Tumorzellen zu einer Tumorhäufigkeit von 66,7% führte, d.h. bei zwei von drei Mäusen entwickelte sich ein palpabler Tumor. Der Durchmesser des Tumors wurde täglich gemessen und mit einem Wert von 0,1 cm für die Hautdicke des Tieres korrigiert. Das Volumen wurde unter der Annahme einer kugelförmigen Gestalt des Tumors berechnet. Die Mäuse wurden am Tag 120 oder, falls der Tumor einen Durchmesser von mehr als 1,8 cm erreicht hatte, früher geopfert. Die 80 Tiere wurden in vier Gruppen (I bis IV) mit je 20 Mäusen unterteilt. Am Tag Null wurde allen Tieren eine Suspension von 2,5 x 10³ Tumorzellen inokuliert.

Die Tiere in der Gruppe I (Kontrollgruppe) erhielten ihr normales Futter ohne Zusatz des Peptidgemisches. Die Entwicklung des Tumors war bei positiv reagierenden Tieren erfahrungsgemäß ab der vierten Woche nach der Inokulation messbar. Am Tag 50 nach der Inokulation hatte sich bei insgesamt 14 Tieren dieser Gruppe ein gut messbarer Tumor entwickelt. Die Tumorinzidenz am Tag 50 lag also bei 70%. Das Durchschnittvolumen des Tumors bei den positiven Tieren wurde wie folgt bestimmt:

| | |
|---|---|
| Tag 50 | 0,81 cm³ |
| Tag 60 | 1,23 cm³ |
| Tag 70 | 1,74 cm³ |

Die Tiere der Gruppe II erhielten ab dem ersten Tag der Inokulation täglich 0,4 µg des erfindungsgemäßen Peptidgemisches zu ihrem Futter zugemischt. Am Tag 50 nach der Inokulation war nur bei 4 Tieren ein palpabler Tumor gut messbar. Die Tumorinzidenz am Tag 50 lag also bei 25%. Das Durchschnittvolumen des Tumors bei den positiven Tieren wurde wie folgt bestimmt:

| | |
|---|---|
| Tag 50 | 0,32 cm³ |
| Tag 60 | 0,57 cm³ |
| Tag 70 | 0,71 cm³ |

Bei den Tieren der Gruppe III wurde die Behandlung mit dem Peptidgemisch erst ab dem 7. Tag nach der Inokulation begonnen. Es wurde eine tägliche Dosis von 0,4 µg Peptidgemisch pro Tier zum Futter zugemischt. Am Tag 50 nach der Inokulation war bei insgesamt 9 Tieren ein palpabler Tumor gut identifizierbar. Die Tumorinzidenz am Tag 50 lag also bei 45,0%. Das Durchschnittvolumen des Tumors bei den positiven Tieren wurde wie folgt bestimmt:

| | |
|---|---|
| Tag 50 | 0,48 cm³ |
| Tag 60 | 0,77 cm³ |
| Tag 70 | 0,93 cm³ |

Die erfindungsgemäßen Peptide bewirken also eine signifikante Hemmung der malignen Erkrankungsrate der Tiere und eine Regression der Tumorentwicklung.

### Beispiel 6: Wirkung der Peptide auf murinen peritonealen Makrophagen

Verwendet wurden von CD1 Mäusen gewonnene murine peritoneale Makrophagen. Eine peritoneale Spülung wurde unter Verwendung von 5 ml PBS durchgeführt. Nach drei Waschvorgängen mit RPMI 1640 wurden die Zellen auf eine 96-Loch-Kulturplatte mit einer Konzentration von 1x10⁶ Zellen/ml (200 µl/Loch) in 10% FCS-RPMI 1640 Medium gegeben und für 24 Stunden stehen gelassen. Nach der Inkubation wurden die Zellen dreimal mit RPMI 1640 gewaschen und in 10% FCS-RPMI 1640 Medium (100 µl/Loch) in Abwesenheit (unstimuliert) oder in Anwesenheit (stimuliert) verschiedener Stimulantien resuspendiert. Nach 24 Stunden Inkubation wurden NO, IL-10 und TNF-α im Kulturüberstand gemessen.

| **Probe** | **NO** | **TNF-α** | **IL-10** | **Überlebensrate** |
|---|---|---|---|---|
| | **(µm)** | **(pg/ml)** | **(pg/ml)** | **(%)** |
| Kontrolle | 1.1 | 0 | 0 | 100 |
| HT-C 5 µg/ml | 0.7 | 244 | 6 | 80.3 |
| HT-C 100 µg/ml | 0.8 | 312 | 26 | 10.7 |
| HT-D 5 µg/ml | 0.8 | 18 | 0 | 87.6 |
| HT-D 100 µg/ml | 1.3 | 0 | 0 | 42.8 |

Das Peptid HT-C zeigt eine deutlich stärkere zytotoxische Wirkung als HT-D gegenüber der hier untersuchten peritonealen Makrophagen aus Mäuse.
Die untersuchten Peptide induzieren keine NO der murinen peritonealen Makrophagen.

### Beispiel 7: Wirkung der Peptide als solche und im Gemisch mit Kohlensuboxid Verbindung (MCS-18) auf Krebszellen der Linie COLO-205

**Methoden:**
Zelllinie: Darmkrebszelllinie: Colo 205 (ATCC Nr. CCL-222), Organismus. Homo sapiens, Literatur: Cancer Res., 38, 1345-1355, 1978. PNAS, 99, 10718-10723, 2002.
Nach Adherenz der Zellen (Inkubation 24 h, Zellzahl 1x10⁴ Zellen pro Ansatz) wurden die Zellen mit MCS und/oder Peptiden (CZT, DZT) 24 h behandelt und anschließend der Prozentsatz lebender Zellen mittels MTT-Test bestimmt.
Zelllinie: Lungenkarzinomzelllinie: A549 (ATCC Nr. CCL-185), Organismus: Homo sapeins
Literatur: Giard DJ, et al.. J. Natl. Cancer Inst. 51: 1417-1423, 1973.
Nach Adherenz der Zellen (Inkubation 24 h, Zellzahl 5x10³ Zellen pro Ansatz) wurden die Zellen mit MCS und/oder Peptiden (CZT, DZT) 72 h behandelt und anschließend der Prozentsatz lebender Zellen mittels AlamarBlue-Test bestimmt.

**MTT-Test:**
Der MTT-Zellwachstums-Test wurde nach Alley et. al. (M.C. Alley et al., Proc. Am. Assoc. Cancer Res., 27:389, 1986; M.C. Alley et al., Cancer Res. 48:589-601, 1988) durchgeführt. Finale Konzentration von 3-(4,5-Dimethylthiazol-2-yl)-2,5-diphenyltetrazoliumbromid war 0,4 mg/ml).
AlamarBlue^{™}-Zellwachstums-Test wurde nach Angaben des Herstellers (Serotec, Oxford, England, www.serotc.com) durchgeführt.

### Resultat:

Wie in Tabelle 2 gezeigt wird in Gegenwart der Peptide CZT und DZT das Wachstum der Colo 205 Zellen bereits nach 24 Stunden bei der Konzentration 100 µg/ml stark gehemmt. Die Anzahl der lebenden Zellen beträgt nur noch ca. 50%. Wird zusätzlich zum Peptid CZT die Substanz MCS 18 gegeben, kann noch verbesserte hemmende Wirkung des Peptids beobachtet werden. MCS18 selbst hat bei 100 mg/ml keinerlei hemmende Wirkung auf das Wachstum der Colo 205 Zellen (Tabelle 3). Somit kann die Kombination von CZT Peptid und MCS eine erhöhte Anti-Tumor-Aktivität bei Darmkrebs führen.

Die Peptide BZT, CZT und DZT konnten auch das Wachstum von Lungenkarzinomzelllinien bei einer Konzentration von 100 µg/ml hemmen (siehe Figur 4). Es konnten nur noch ca. 50% lebende Zellen nach 72 h bestimmt werden.

**Tabelle 2:**

| Substanz | Konzentration in [µg/ml] | Lebende Colo 205 Zellen in [%] |
|---|---|---|
| - | - | 100 |
| CZT Peptid | 50 | 92,15 |
| CZT Peptid | 100 | 48,36 |
| DZT Peptid | 50 | 92,15 |
| DZT Peptid | 100 | 58,66 |

**Tabelle 3:**

| Substanz | Konzentration in [µg/ml] | Lebende Colo 205 Zellen in [%] |
|---|---|---|
| - | - | 100 |
| MCS 18 | 100 | 100 |
| CZT Peptid | 50 | 92,15 |
| CZT Peptid + | 50 | 66,66 |
| MCS18 | 100 | |

## Patentansprüche

1. Cysteinhaltige Peptide der Struktur 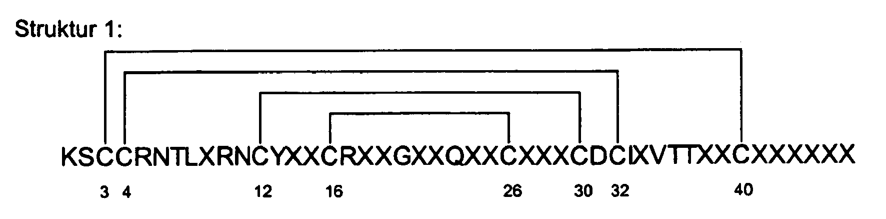 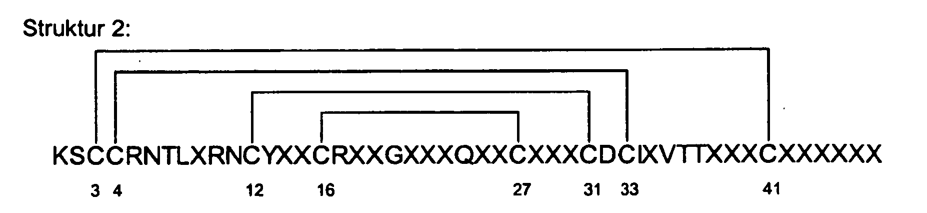 welche die natürlichen Abwehrkräfte pflanzlicher, tierischer oder menschlicher Organismen gegen bakterielle, fungale, virale oder andere Pathogene unterstützen, wobei die durchgezogenen Linien die Disulfidbrücken symbolisieren und wobei X unabhängig voneinander jede beliebige natürlich vorkommende Aminosäure darstellt.

2. Cysteinhaltige Peptide nach Anspruch 1, wobei an Position 15 die Aminosäure G und/oder an Position 19 die Aminosäure T und/oder an Position 27 die Aminosäure Q und/oder an Position 28 die Aminosäure R und/oder an Position 34 die Aminosäure H und/oder an Position 38 die Aminosäure T und/oder an Position 43 die Aminosäure S und/oder an Position 44 die Aminosäure H und/oder an Position 46 die Aminosäure S ist.

3. Cysteinhaltige Peptide nach einem der vorhergehenden Ansprüche, nämlich
KSCCRNTLGRNCYNGCRFTGGSQPTCGRLCDCIHVTTTTCPSSHPS (Hellethionin-A),
KSCCRNTLGRNCYNACRFTGGSQPTCGRLCDCIHVTTTTCPSSHPS (Hellethionin-B1),
KSCCRNTLARNCYNACRFTGGSQPTCGRLCDCIHVTTTTCPSSHPS (Hellethionin-B2),
KSCCRNTLGRNCYNACRLPGTPGIPTCATLCDCIHVTTPTCPSSHPR (Hellethionin-B3),
KSCCRNTLARNCYNACRFTGTSQPYCARLCDCIHVTTPTCPSSHPR (Hellethionin-B4),
KSCCRNTLARNCYNACRFTGGSQPTCATLCDCIHVTTPTCPSSHPR (Hellethionin-B5),
KSCCRNTLARNCYNVCRFGGGSQAYCARFCDCIHVTTSTCPSSHPS (Hellethionin-B6),
KSCCRNTLGRNCYNACRLTGTSQATCATLCDCIHVTATTCRPPYPS (Hellethionin-C),
KSCCRNTLARNCYNACRFTGGSQPTCGILCDCIHVTTTTCPSSHPS (Hellethionin-D),
KSCCRNTLGRNCYAACRLTGLFSQEQCARLCDCITVTTPTPCPRTHPS (Hellethionin-E1),
KSCCRNTLGRNCYAACRLTGTFSQEQCARLCDCITVTTPTPCPRTHPS (Hellethionin-E2).

4. Nukleinsäuresequenz, die ein cysteinhaltiges Peptid nach einem der vorhergehenden Ansprüche codiert.

5. RNA-Sequenz nach Anspruch 4.

6. DNA-Sequenz nach Anspruch 4.

7. Ester-Derivate, Amid-Derivate, Halogen-Derivate, Methyl-Derivate, Salz-Derivate, cyclische Derivate und Derivate mit einem modifizierten Rückgrat der cysteinhaltigen Peptide nach einem der Ansprüche 1 bis 3.

8. DNA-Vektor oder DNA-Konstrukt, der eine DNA-Sequenz nach Anspruch 6 enthält.

9. Monoklonale Antikörper gerichtet gegen ein Epitop der cysteinhaltigen Peptide nach einem der Ansprüche 1 bis 3.

10. Verwendung von cysteinhaltigen Peptiden nach einem der Ansprüche 1 bis 3 und Gemische dieser cysteinhaltigen Peptide zur Herstellung einer pharmazeutischen Formulierung zur Behandlung von Krankheiten.

11. Verwendung von cysteinhaltigen Peptiden nach einem der Ansprüche 1 bis 3 und Gemische dieser cysteinhaltigen Peptide zur Herstellung einer pharmazeutischen Formulierung zur Behandlung von durch Pathogene verursachte Krankheiten, zur Behandlung von durch Bakterien, Fungi oder Viren verursachte Krankheiten, zur Behandlung von Krankheiten bei Mensch und Tier, insbesondere bei Pferden, zur Behandlung von Krankheiten, die durch fehlerhafte Bioregulation des Immunsystems verursacht sind oder von einer fehlerhaften Bioregulation des Immunsystems begleitet werden, zur Behandlung von Autoimmunkrankheiten, zur Behandlung von Krebserkrankungen sowie zur Behandlung von AIDS.

12. Cysteinhaltige Peptide nach einem der Ansprüche 1 bis 3, Gemische dieser cysteinhaltigen Peptide und/oder pharmazeutisch akzeptable Salze dieser cysteinhaltigen Peptide zur Herstellung einer pharmazeutischen Formulierung für die Behandlung von Krankheiten.

13. Cysteinhaltige Peptide nach einem der Ansprüche 1 bis 3, Gemische dieser cysteinhaltigen Peptide und/oder pharmazeutisch akzeptable Salze dieser cysteinhaltigen Peptide zur Herstellung einer pharmazeutischen Formulierung für die Behandlung von durch Pathogenen verursachten Krankheiten sowie zur Herstellung einer pharmazeutischen Formulierung für die Prophylaxe und/oder Behandlung von Krebs.

14. Cysteinhaltige Peptide nach Anspruch 13, wobei es sich bei dem Krebs um einen Krebs ausgewählt aus der Gruppe umfassend Aderhautmelanom, Akute Leukämie, Akustikusneurinom, Ampullenkarzinom, Analkarzinom, Astrozytom, Basaliom, Bauchspeicheldrüsenkrebs, Blasenkrebs, Bronchialkarzinom, Brustkrebs, Burkitt-Lymphom, Corpuskarzinom, CUP-Syndrom, Dickdarmkrebs, Dünndarmkrebs, Dünndarmtumore, Eierstockkrebs, Endometriumkarzinom, Ependymom, Epithel-Krebsarten, Ewing-Tumoren, Gastrointestinale Tumoren, Gallenblasenkrebs, Gebärmutterkrebs, Gebärmutterhalskrebs, Glioblastom, Gynäkologische Tumoren, Hals-, Nasen- und Ohrentumoren, Hämatologische Neoplasien, Haarzell-Leukämie, Harnröhrenkrebs, Hautkrebs, Hirntumoren (Gliome), Hirnmetastasen, Hodenkrebs, Lymphdrüsenkrebs (Hodgkin/Non-Hodgkin), Hypophysentumor, Karzinoide, Kaposi-Sarkom, Kehlkopfkrebs, Keimzellentumor, Knochenkrebs, kolorektales Karzinom, Kopf-Hals-Tumore, Kopf- und Nackenkrebs, Kolonkarzinom, Kraniopharyngeome, Krebs im Mundbereich und auf der Lippe, Krebs des zentralen Nervensystems, Leberkrebs, Lebermetastasen, Leukämie, Lidtumor, Lungenkrebs, Lymphome, Magenkrebs, Malignes Melanom, Mammakarzinom, Mastdarmkrebs, Medulloblastome, Melanom, Meningeome, Morbus Hodgkin, Mycosis fungoides, Nasenkrebs, Neurinom, Nierenkrebs, Non-Hodgkin-Lymphome, Oligodendrogliom, Ösophaguskarzinom, Osteosarkom, Ovarial-Karzinom, Pankreaskarzinom, Peniskrebs, Plasmozytom, Prostatakrebs, Rachenkrebs, Rektumkarzinom, Retinoblastom, Scheidenkrebs, Schilddrüsenkarzinom, Schneeberger Krankheit, Speiseröhrenkrebs, Spinaliom, T-Zell-Lymphom (Mycosis fungoides), Thymom, Tubenkarzinom, Tumoren des Auges, Urethrakrebs, Urologische Tumoren, Urothelkarzinom, Vulvakrebs, Warzenbeteiligung, Weichteiltumoren, Wilms Tumor, Zervixkarzinom und Zungenkrebs handelt.

15. Pharmazeutische Zubereitung, umfassend ein oder mehrere cysteinhaltige Peptide nach den Ansprüchen 1 bis 3.

16. Pharmazeutische Zubereitung nach Anspruch 15, umfassend ein oder mehrere cysteinhaltige Peptide nach den Ansprüchen 1 bis 3 und wenigstens ein Kohlensuboxid-Derivat.

17. Pharmazeutische Zubereitung nach Anspruch 15 oder 16, enthaltend ein oder mehrere cysteinhaltige Peptide nach den Ansprüchen 1 bis 3 und wenigstens eine zytostatisch und/oder zytotoxisch wirkende Verbindung.

18. Verfahren zur Gewinnung der cysteinhaltigen Peptide nach den Ansprüchen 1 bis 3 durch Extraktion aus der Pflanze Helleborus species.

19. Verfahren nach Anspruch 18, wobei als erster Schritt des Verfahrens eine Entfettung des Pflanzenmaterials unter Verwendung nicht-polarer Lösungsmittel erfolgt, insbesondere unter Verwendung von tert.-Butylmethylether.

20. Verfahren zur Gewinnung der cysteinhaltigen Peptide nach den Ansprüchen 1 bis 3 durch gentechnologische Methoden.

21. Verfahren nach Anspruch 20, wobei die Thionin-Gene eines Thionin-produzierenden Getreides durch die Thionin-Gene der Pflanze Helleborus species ersetzt werden.

22. Verfahren zur synthetischen Gewinnung der cysteinhaltigen Peptide nach den Ansprüchen 1 bis 3 durch Peptid-Synthese.

## Claims

1. Peptides containing cysteine an having the following structure 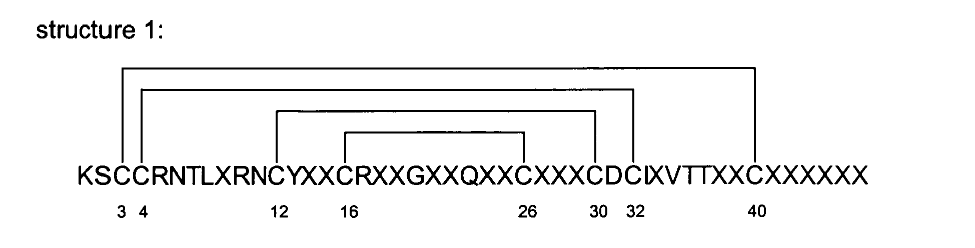 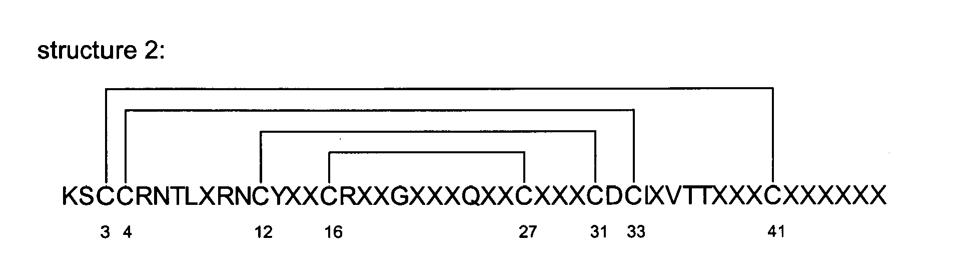 and supporting the natural immune defences of plants and animal or human organisms in combating bacterial, fungal, viral or other pathogens, wherein the continuous lines represent the disulfide bridges and wherein X independently represents any natural amino acid.

2. Peptides containing cysteine according to claim 1, wherein the amino acid G is located at position 15 and/or the amino acid T is located at position 19 and/or the amino acid Q is located at position 27 and/or the amino acid R is located at position 28 and/or the amino acid H is located at position 34 and/or the amino acid T is located at position 38 and/or the amino acid S is located at position 43 and/or the amino acid H is located at position 44 and/or the amino acid S is located at position 46.

3. Peptides containing cysteine according to any one of the preceding claims, namely
KSCCRNTLGRNCYNGCRFTGGSQPTCGRLCDCIHVTTTTCPSSHPS (hellethionin A),
KSCCRNTLGRNCYNACRFTGGSQPTCGRLCDCIHVTTTTCPSSHPS (hellethionin B1),
KSCCRNTLARNCYNACRFTGGSQPTCGRLCDCIHVTTTTCPSSHPS (hellethionin B2),
KSCCRNTLGRNCYNACRLPGTPQPTCATLCDCIHVTTPTCPSSHPR (hellethionin B3),
KSCCRNTLARNCYNACRFTGTSQPYCARLCDCIHVTTPTCPSSHPR (hellethionin B4),
KSCCRNTLARNCYNACRFTGGSQPTCATLCDCIHVTTPTCPSSHPR (hellethionin B5),
KSCCRNTLARNCYNVCRFGGGSQAYCARFCDCIHVTTSTCPSSHPS (hellethionin B6),
KSCCRNTLGRNCYNACRLTGTSQATCATLCDCIHVTATTCRPPYPS (hellethionin C),
KSCCRNTLARNCYNACRFTGGSQPTCGILCDCIHVTTTTCPSSHPS (hellethionin D),
KSCCRNTLGRNCYAACRLTGLFSQEQCARLCDCITVTTPTPCPRTHPS (hellethionin E1),
KSCCRNTLGRNCYAACRLTGTFSQEQCARLCDCITVTTPTPCPRTHPS (hellethionin E2)

4. Nucleic acid sequence encoding a peptide containing cysteine according to any one of the preceding claims.

5. RNA sequence according to claim 4.

6. DNA sequence according to claim 4.

7. Ester derivatives, amide derivatives, halogen derivatives, methyl derivatives, salt derivatives, cyclic derivatives and derivatives having a modified backbone, of the peptides containing cysteine according to any one of claims 1 to 3.

8. DNA vector or DNA construct including a DNA sequence according to claim 6.

9. Monoclonal antibodies directed against an epitope of the peptides containing cysteine according to any one of claims 1 to 3.

10. Use of peptides containing cysteine according to any one of claims 1 to 3 and mixtures of said peptides containing cysteine for the preparation of a pharmaceutical formulation for the treatment of diseases.

11. Use of peptides containing cysteine according to any one of claims 1 to 3 and mixtures of said peptides containing cysteine for the preparation of a pharmaceutical formulation for the treatment of diseases caused by pathogens; for the treatment of diseases caused by bacteria, fungi or viruses; for the treatment of diseases in humans and animals, especially in horses; for the treatment of diseases caused by defective bioregulation of the immune system or accompanied by a defective bioregulation of the immune system; for the treatment of autoimmune diseases; for the treatment of cancers; and for the treatment of AIDS.

12. Peptides containing cysteine according to any one of claims 1 to 3, mixtures of said peptides containing cysteine and/or pharmaceutically acceptable salts of said peptides containing cysteine for the preparation of a pharmaceutical formulation for the treatment of diseases.

13. Peptides containing cysteine according to any one of claims 1 to 3, mixtures of said peptides containing cysteine and/or pharmaceutically acceptable salts of said peptides containing cysteine for the preparation of a pharmaceutical formulation for the treatment of diseases caused by pathogens as well as for the preparation of a pharmaceutical formulation for the prophylaxis and/or treatment of cancer.

14. Peptides containing cysteine according to claim 13, wherein the cancer is a cancer selected from the group comprising: choroidal melanoma, acute leukemia, acoustic neurinoma, ampullary carcinoma, anal carcinoma, astrocytoma, basal cell carcinoma, pancreatic cancer, bladder cancer, bronchial carcinoma, breast cancer, Burkitt's lymphoma, corpus cancer, CUP syndrome, cancer of the large intestine, cancer of the small intestine, tumours of the small intestine, ovarian cancer, endometrial carcinoma, ependymoma, epithelial cancers, Ewing tumours, gastrointestinal cancers, gall bladder cancer, uterine cancer, cervical cancer, glioblastoma, gynaecological cancers, tumours of ear, nose and throat, haematological neoplasias, hairy cell leukemia, urethral cancer, skin cancer, brain tumours (gliomas), brain metastases, testicular cancer, lymph node cancer (Hodgkin/Non-Hodgkin), hypophysis tumour, carcinoids, Kaposi's sarcoma, laryngeal cancer, germ cell tumour, bone cancer, colorectal carcinoma, head and neck tumours, head and neck cancers, colon carcinoma, craniopharyngiomas, cancer in the area of the mouth and on the lip, cancer of the central nervous system, liver cancer, liver metastases, leukemia, tumour of the eyelid, lung cancer, lymphomas, stomach cancer, malignant melanoma, breast carcinoma, rectal cancer, medulloblastomas, melanoma, meningiomas, Hodgkin's disease, mycosis fungoides, nose cancer, neurinoma, kidney cancer, Non-Hodgkin's lymphomas, oligodendroglioma, oesophageal carcinoma, osteosarcoma, ovarian carcinoma, pancreatic carcinoma, penile carcinoma, plasmacytoma, prostate carcinoma, throat cancer, rectal carcinoma, retinoblastoma, vaginal cancer, thyroid carcinoma, Schneeberg lung cancer, oesophageal cancer, spinocellular carcinoma, T-cell lymphoma (mycosis fungoides), thymoma, tube carcinoma, eye tumours, urological tumours, urothelial carcinoma, vulvar carcinoma, wart appearance, soft tissue tumours, Wilm's tumor and tongue cancer.

15. Pharmaceutical preparation comprising one or more peptides containing cysteine according any one of claims 1 to 3.

16. Pharmaceutical preparation according to claim 15, comprising one or more peptides containing cysteine according to claims 1 to 3 and at least one carbon suboxide derivative.

17. Pharmaceutical preparation according to claim 15 or 16, containing one or more peptides containing cysteine according to claims 1 to 3 and at least one compound having cytostatic and/or cytotoxic activity.

18. Method for obtaining said peptides containing cysteine according to any one of claims 1 to 3 by means of extraction form the plant species helleborus.

19. Method according to claim 18, wherein the first step of the method consists of defatting of the plant material using non-polar solvents, especially using tert. butyl methyl ether.

20. Method for obtaining the peptides containing cysteine according to claims 1 to 3 by using methods from the field of gene technology.

21. Method according to claim 20, wherein the thionine genes of a grain producing thionine are replaced by the thionine genes of the plant species helleborus.

22. Method for the synthetic production of the peptides containing cysteine according to claims 1 to 3 by using peptide synthesis.

## Revendications

1. Des peptides contenant des cystéines selon la structure suivante : 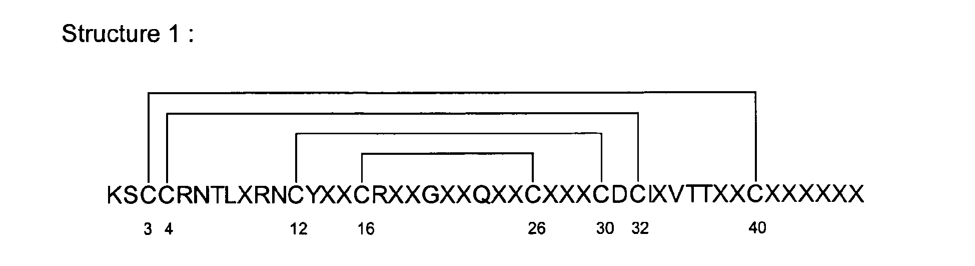 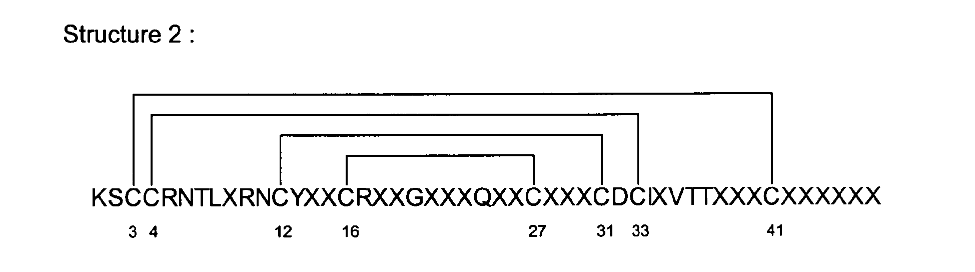 qui soutiennent les défenses immunitaires d'organismes végétaux, animaliers ou humains contre des pathogènes bactériens, fongiques, viraux ou autres, les lignes continues symbolisant les ponts disulfures et X représentant un acide aminé naturel quelconque, l'un indépendamment de l'autre.

2. Les peptides contenant des cystéines selon la revendication 1, l'acide aminé G étant en position 15 et/ ou l'acide aminé T étant en position 19 et/ ou l'acide aminé Q étant en position 27 et/ ou l'acide aminé R étant en position 28 et/ ou l'acide aminé H étant en position 34 et/ ou l'acide aminé T étant en position 38 et/ ou l'acide aminé S étant en position 43 et/ ou l'acide aminé H étant en position 44 et/ ou l'acide aminé S étant en position 46.

3. Les peptides contenant des cystéines selon l'une quelconque des revendications précédentes, à savoir
KSCCRNTLGRNCYNGCRFTGGSQPTCGRLCDCIHVTTTTCPSSHPS (helléthionine-A),
KSCCRNTLGRNCYNACRFTGGSQPTCGRLCDCIHVTTTTCPSSHPS (helléthionine-B1),
KSCCRNTLARNCYNACRFTGGSQPTCGRLCDCIHVTTTTCPSSHPS (helléthionine-B2),
KSCCRNTLGRNCYNACRLPGTPQPTCATLCDCIHVTTPTCPSSHPR (helléthionine-B3),
KSCCRNTLARNCYNACRFTGTSQPYCARLCDCIHVTTPTCPSSHPR (helléthionine-B4),
KSCCRNTLARNCYNACRFTGGSQPTCATLCDCIHVTTPTCPSSHPR (helléthionine-B5),
KSCCRNTLARNCYNVCRFGGGSQAYCARFCDCIHVTTSTCPSSHPS (helléthionine-B6),
KSCCRNTLGRNCYNACRLTGTSQATCATLCDCIHVTATTCRPPYPS (helléthionine-C),
KSCCRNTLARNCYNACRFTGGSQPTCGILCDCIHVTTTTCPSSHPS (helléthionine-D),
KSCCRNTLGRNCYAACRLTGLFSQEQCARLCDCITVTTPTPCPRTHPS (helléthionine-E1),
KSCCRNTLGRNCYAACRLTGTFSQEQCARLCDCITVTTPTPCPRTHPS (helléthionine-E2).

4. La séquence d'acide nucléique qui code un peptide contenant des cystéines selon l'une quelconque des revendications précédentes.

5. Séquence ARN selon la revendication 4.

6. Séquence ADN selon la revendication 4.

7. Des dérivés d'esters, des dérivés d'amides, des dérivés d'halogènes, des dérivés de méthyl, des dérivés de sel, des dérivés cycliques et des dérivés dotés d'une chaîne principale modifiée des peptides contenant des cystéines selon l'une quelconque des revendications 1 à 3.

8. Vecteur ADN ou construction ADN qui contient une séquence ADN selon la revendication 6.

9. Anticorps monoclonal dirigé contre un épitope des peptides contenant des cystéines selon l'une quelconque des revendications 1 à 3.

10. Utilisation des peptides contenant des cystéines selon l'une quelconque des revendications 1 à 3 et mélanges desdits peptides contenant des cystéines destinée à la préparation d'une formulation pharmaceutique pour traiter des maladies.

11. Utilisation des peptides contenant des cystéines selon l'une quelconque des revendications 1 à 3 et mélanges desdits peptides contenant des cystéines destinée à la préparation d'une formulation pharmaceutique pour traiter des maladies provoquées par des pathogènes, pour traiter des maladies provoquées par des bactéries, champignons ou virus, pour traiter des maladies chez l'être humain et l'animal, en particulier chez des chevaux, pour traiter des maladies provoquées par un désordre de la biorégulation du système immunitaire ou accompagnées d'un désordre de la biorégulation du système immunitaire, pour traiter des maladies auto-immunes, pour traiter des maladies cancéreuses ainsi que pour traiter le SIDA.

12. Les peptides contenant des cystéines selon l'une quelconque des revendications 1 à 3, des mélanges desdits peptides contenant des cystéines et/ ou des sels pharmaceutiquement acceptables desdits peptides contenant des cystéines destinés à la fabrication d'une formulation pharmaceutique pour traiter des maladies.

13. Les peptides contenant des cystéines selon l'une quelconque des revendications 1 à 3, des mélanges desdits peptides contenant des cystéines et/ ou des sels pharmaceutiquement acceptables destinés à la préparation d'une formulation pharmaceutique pour traiter des maladies provoquées par des pathogènes ainsi qu'à la préparation d'une formulation pharmaceutique pour prévenir et/ ou traiter le cancer.

14. Les peptides contenant des cystéines selon la revendication 13, le cancer étant un cancer sélectionné parmi le groupe comprenant le suivant : mélanome choroïdien, leucémie aigue, neurinome acoustique, ampullome, cancer anal, astrocytome, basaliome, cancer du pancréas, cancer de la vessie, carcinome bronchique, cancer du sein, lymphome de Burkitt, carcinome de corps de l'utérus, syndrome CAPI *(carcinomes de site primitif inconnu),* cancer du côlon, cancer de l'intestin grêle, tumeurs de l'intestin grêle, cancer de l'ovaire, carcinome de l'endomètre, épendymome, types de cancer épithéliaux, tumeurs d'Ewing, tumeurs gastro-intestinales, cancer de la vésicule biliaire, cancer de l'utérus, cancer du col de l'utérus, glioblastome, tumeurs gynécologiques, des tumeurs de la gorge, du nez et de l'oreille, néoplasies hématologiques, leucémie à tricholeucocytes, cancer de l'urètre, cancer de la peau, tumeurs cérébrales (gliomes), métastases cérébrales, cancer du testicule, cancer des ganglions lymphatiques (hodgkinien/ non hodgkinien), tumeur de l'hypophyse, carcinoïdes, sarcome de Kaposi, cancer du larynx, tumeur germinale, cancer des os, carcinome colorectal, tumeurs de la tête et du cou, cancer de la tête et du cou, carcinome du colon, craniopharyngiome, cancer de la cavité buccale et de la lèvre, cancer du système nerveux central, cancer du foie, métastases du foie, leucémie, tumeur de la paupière, cancer du poumon, lymphomes, cancer de l'estomac, mélanome malin, carcinome mammaire, cancer du rectum, médulloblastomes, mélanome, méningiomes, maladie de hodgkin, mycosis fongoïde, cancer du nez, neurinome, cancer du rein, lymphomes non hodgkiniens, oligodendrogliome, carcinome de l'oesophage, ostéosarcome, carcinome de l'ovaire, carcinome du pancréas, cancer du pénis, plasmocytome, cancer de la prostate, cancer du pharynx, carcinome du rectum, rétinoblastome, cancer du vagin, cancer de la glande thyroïde, maladie de Schneeberg, spinaliome, lymphome à cellules T (mycosis fongoïde), thymome, carcinome des trompes, tumeurs de l'oeil, cancer de l'urètre, tumeurs urologiques, carcinome urothélial, cancer de la vulve, apparition de verrues, tumeurs des parties molles, tumeur de Wilms, carcinome cervicale et cancer de la langue.

15. Préparation pharmaceutique, comprenant un ou plusieurs peptide(s) contenant des cystéines selon les revendications 1 à 3.

16. Préparation pharmaceutique selon la revendication 15, comprenant un ou plusieurs peptide(s) contenant des cystéines selon les revendications 1 à 3 et au moins un dérivé de sous-oxyde de carbone.

17. Préparation pharmaceutique selon la revendication 15 ou 16 contenant un ou plusieurs peptide(s) contenant des cystéines selon les revendications 1 à 3 et au moins un composé à effet cytostatique et/ ou cytotoxique.

18. Procédé pour la préparation de peptides contenant des cystéines selon les revendications 1 à 3 par extraction à partir des plantes étant des espèces du genre hellébore.

19. Procédé selon la revendication 18 le premier pas du procédé étant un dégraissage du matériau végétal en utilisant un solvant non polaire, en particulier en utilisant méthyltertiobutyléther.

20. Procédé pour l'obtention des peptides contenant des cystéines selon les revendications 1 à 3 par des procédés du génie génétique.

21. Procédé selon la revendication 20, les gènes de thionine de grains producteurs de la thionine étant substitués par les gènes de thionine des étant des espèces du genre hellébore.

22. Procédé pour l'obtention par synthèse des peptides contenant des cystéines selon les revendications 1 à 3 par la synthèse peptidique.
